(19) **Europäisches Patentamt**

**European Patent Office**

**Office européen des brevets**

(11) Veröffentlichungsnummer: **0 203 382 B1**

(12) **EUROPÄISCHE PATENTSCHRIFT**

(45) Veröffentlichungstag der Patentschrift: **15.09.93**

(51) Int. Cl.5: **C12P 21/02**, C12N 15/00

(21) Anmeldenummer: **86105722.2**

(22) Anmeldetag: **25.04.86**

Die Akte enthält technische Angaben, die nach
dem Eingang der Anmeldung eingereicht wurden
und die nicht in dieser Patentschrift enthalten sind.

(54) **Verfahren zur Herstellung und Reinigung von alpha-Interferon.**

(30) Priorität: **27.04.85 DE 3515336**

(43) Veröffentlichungstag der Anmeldung:
**03.12.86 Patentblatt 86/49**

(45) Bekanntmachung des Hinweises auf die
Patenterteilung:
**15.09.93 Patentblatt 93/37**

(84) Benannte Vertragsstaaten:
**AT BE CH DE FR GB IT LI LU NL SE**

(56) Entgegenhaltungen:
**EP-A- 0 018 218**
**EP-A- 0 043 980**
**EP-A- 0 108 585**
**DE-A- 3 306 060**
**US-A- 4 364 863**

(73) Patentinhaber: **BOEHRINGER INGELHEIM IN-
TERNATIONAL GmbH**

**D-55216 Ingelheim(DE)**

(72) Erfinder: **Bodo, Gerhard, Prof. Dr.
Belghofergasse 27
A-1120 Wien(AT)**
Erfinder: **Maurer-Fogy, Ingrid, Dr.
Mariannengasse 24/2/20
A-1090 Wien(AT)**
Erfinder: **Falkner, Edgar, Dr.
Strohberggasse 9
A-1120 Wien(AT)**
Erfinder: **Lindner, Silvia Jutta, Dr.
Sossenstrasse 6
A-2380 Perchtoldsdorf(AT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung ist ein Verfahren zur Herstellung eines hochreinen, nichtimmunogenen, antiviral und immunregulatorisch wirksamen, homogenen $\alpha$-Interferons, das Protein selbst sowie dessen Verwendung.

Interferone sind körpereigene Proteine, die in den verschiedensten Spezies nachgewiesen werden können. Die ihnen eigenen antiviralen und immunregulatorischen Eigenschaften haben sie schon frühzeitig als geeignet für unterschiedlichste Einsatzgebiete erscheinen lassen. Untersuchungen haben gezeigt, daß es verschiedene Interferon-Klassen gibt. Neben den Interferonen $\alpha$, $\beta$ und $\gamma$ wurde vor kurzem das Interferon-$\omega$ gefunden und seine Struktur aufgeklärt.

Die in die Interferone gesteckten hohen Erwartungen als wirksames Mittel gegen virale Erkrankungen und gegen Krebs haben schon früh zu Untersuchungen mit Interferonpräparaten aus natürlichem Material geführt, bei denen jedoch gravierende Nebenwirkungen auftraten. Die bei diesen Unterschungen eingesetzten Präparate enthielten selbst nach aufwendigster Reinigung komplexe Mischungen verschiedener Interferone und in vielen Fällen auch anderer Proteine. Die Ursache liegt darin, daß es bei einigen der Interferone Subtypen gibt, die sich mehr oder weniger voneinander unterscheiden; so sind beispielsweise vom Interferon-$\alpha$ mittlerweile mehr als 20 verschiedene Typen bekannt.

Erst durch die Herstellung der Interferone durch gentechnologische Verfahren ließen sich typenreine Interferonpräparate untersuchen.

Hierzu zählen auch die in den klinischen Prüfungen eingesetzten rekombinanten Interferone-$\alpha_2$ (auch $\alpha$ A genannt) Ausschlaggebende Bedeutung bei der Herstellung menschlicher Proteine durch Mikroorganismen kommt der Reinigung der Proteine zu. Jede Beimengung, die aus dem Wirtsorganismus stammt, würde beim Einsatz des Produktes am Menschen zu Immunabwehrreaktionen führen, die lebensbedrohend sein können. Die Abtrennung von Beimengungen dieser Art bereitet heutzutage jedoch kaum Probleme und die mittlerweile äußerst empfindlichen analytischen Methoden lassen Endotoxine in geringsten Konzentrationen nachweisen. Auch auf dem Gebiet der Interferonforschung wurden Reinigungsverfahren entwickelt, die es ermöglichen, Interferonpräparate zu erhalten, die so gut wie keine Endotoxine mehr enthalten. Beispielhaft genannt sei hier die Arbeit von Staehelin et al. J. Biol. Chem. 256, 9750 (1981).

Alle zur klinischen Prüfung eingesetzten rek. $\alpha$-Interferone sind praktisch Endotoxin-frei.

Umso überraschender war daher das Auftreten von Nebenwirkungen, die sogar zum Abbruch der Interferonbehandlung zwangen. Einige rek. $\alpha$-Interferone erwiesen sich überraschenderweise als immunogen. Antikörper gegen das Interferon waren stimuliert worden.

(Quesada et al. J. Natl. Cancer Inst. 70, No. 6, 1041-1046 (1983); Protzman et al. J. Immunol. Methods 75, 317 - 323 (1984)).

Diese Antikörper können zu bedrohlichen Auswirkungen führen, wenn sie die Wirkung des Interferons beeinflussen. Sie wirken dann nämlich nicht nur auf das rek. $\alpha$-Interferon ein, sondern da das rek. $\alpha$-Interferon identisch mit dem körpereigenen Interferon ist, in gleichem Maße auch auf das körpereigene Interferon.

Verhängnisvoll daran ist, daß diese Antikörper auch nach der Beendigung der Interferon-Behandlung weiterwirken, den Krankheitsverlauf verschlechtern können, die körpereigene Abwehr gegen Virusinfektionen schwächen und den Organismus damit anfälliger für weitere Infektionen werden lassen.

Diese Auswirkungen wurden bereits im Tierversuch bestätigt. Im Sinne einer maximalen Arzneimittelsicherheit ist daher zu fordern, daß das rek.-Interferon-$\alpha_2$ typenrein, praktisch Endotoxin-frei und nicht zuletzt nichtimmunogen sein muß.

Die Aufgabe der vorliegenden Erfindung bestand also darin, ein Verfahren zur Herstellung eines nichtimmunogenen, antiviral und immunregulatorisch wirksamen rek. $\alpha$-Interferons zu entwickeln.

Die Ursache der Immunogenität der genannten $\alpha$-Interferone ist nicht bekannt. Ausgeschlossen werden kann lediglich, daß endotoxische Beimengungen hierfür verantwortlich sind.

Die zur Prüfung eingesetzten Präparate unterscheiden sich primär durch geringfügige Variationen in ihrer Aminosäuresequenz.

|  | Aminosäure 23 | Aminosäure 34 |
|---|---|---|
| Präparat I: | Lysin | Histidin |
| Präparat II: | Arginin | Histidin |
| Präparat III: | Arginin | Arginin |

Neben den strukturellen Unterschieden in der Primärstruktur der klinisch eingesetzten Proteine ist bekannt, daß es sich bei gentechnologisch hergestellten $\alpha$-Interferonen stets um eine Mischung von monomeren, niedermolekularen, reduzierten und oligomeren Formen des Interferons handelt (s. z.B. EPA 108 585, 110 302 und 118 808). Einige dieser Formen zeigen in vitro gleiche, andere aber auch verminderte Wirkungen und einigen werden mögliche immunogene Eigenschaften nachgesagt (s. EPA 108 585 und 110 302).

Beschrieben sind in diesen Patentanmeldungen Verfahren, um diese Interferonformen abzutrennen.

In der EPA 108 585 wird ein Verfahren zur Abtrennung eines "Slow moving monomers" und von Oligomeren beschrieben, bei dem die Interferonprobe bei einem pH von 3 bis 5 einige Zeit bei einer Temperatur von 28-40 °C inkubiert wird.

Die EPA 110 302 beschreibt ein Verfahren, bei dem aus den Oligomeren durch Reduktion mit einem Redox-System das Monomere gebildet wird.

In der EPA 118 808 schließlich wird rekombinantes Interferon-$\alpha$ mit Hilfe von Metallchelatharzen von den oligomeren Formen gereinigt.

Die nach diesen Verfahren erhaltenen Interferone sollen in fast quantitativer Form monomeres Interferon enthalten; Immunogenitätsprüfungen liegen jedoch nicht vor.

Eingehende eigene analytische Untersuchungen haben gezeigt, daß es sich bei rekombinant hergestellten $\alpha$-Interferonen um eine Mischung aus verschiedenen Interferonformen in wechselnden Konzentrationen handelt.

Dazu zählen Oligomere, Tetramere, Trimere und Dimere des Interferons, Methionin-Interferon, reduzierte Formen und Bruchstücke des Interferons und überraschenderweise auch verschiedene monomere Formen des Interferons.

Bei den Oligomeren handelt es sich um $\alpha$-Interferone mit einem Molekulargewicht > 70.000, bei den reduzierten $\alpha$-Interferonen um das Protein mit freien SH-Gruppen und bei dem Methionin-Interferon um ein $\alpha$-Interferon, das am N-terminalen Ende zusätzlich ein Methionin (bedingt durch die mikrobiologische Herstellung des $\alpha$-Interferons)trägt.

Die verschiedenen monomeren Formen stellen, so hat die Analyse gezeigt, verschiedene S-S-Isomere des $\alpha$-Interferons dar.

Um diese Isomeren sprachlich zu unterscheiden, wird im folgenden der Ausdruck nicht-natives Monomer für die Isomeren des hauptsächlich auftretenden $\alpha$-Interferon-Monomers verwendet und dieses als nativ monomeres $\alpha$-Interferon bezeichnet. Damit soll jedoch nicht ausgeschlossen werden, daß es die nicht-nativen Monomeren ebenfalls auch im "natürlichen" Material geben kann.

Bei einem E. coli Fermentationsansatz zur Herstellung von Interferon-$\alpha_2$ ließen sich zum Beispiel mindestens 7 verschiedene Interferonkomponenten nachweisen (K 1 - K 7).

Die Analyse ergab, daß es sich hierbei um Oligomere, Di-/Trimere, Methionin-Interferon, reduzierte Interferone und um ein S-S-Isomeres des nativ-monomeren Interferon-$\alpha_2$, das je eine Disulfidbrücke zwischen den Aminosäuren an Position 1 und 98 und 29 und 138 aufweist (s. auch Wetzel et al. J. Interferon Res., Vol. 1, No. 3, 381 - 391 (1981), handelte.

Wie bereits oben ausgeführt, ist die Ursache der Immunogenität des rek. $\alpha$-Interferons nicht bekannt. Naheliegend ist jedoch auch, daß alle die Formen des $\alpha$-Interfeons immunogen wirken, die sich von den körpereigenen unterscheiden. Hierzu zählen auch die niedermolekularen, die Dimeren und Oligomeren und auch die nicht-nativen Monomere, die anders geknüpfte Disulfidbrücken enthalten.

Solange die Ursachen der Immunogenität noch nicht geklärt sind, sollten bei den Verfahren zur Herstellung von rek. $\alpha$-Interferonen keine Bedingungen verwendet werden, die die Bildung dieser, in ihrer Auswirkung unbekannten Formen fördern könnten. Das heißt, daß auch die Reinigung des Interferons unter schonendsten, die Nativität nicht gefährdenden Bedingungen durchgeführt werden sollte. Erhöhte Temperatur und Reduktionsmittel zählen nicht zu diesen Bedingungen.

Selbstverständlich ist bei allen Reinigungsschritten zu gewährleisten, daß keine Fremdstoffe eingeschleppt werden, beispielweise bei der Verwendung von Metallchelatharzen oder ähnlich problematischen

3

Reagenzien.

Gegenstand der Erfindung ist ein Verfahren zur Herstellung von rekombinantem α-Interferon, gekennzeichnet durch die im Hauptanspruch aufgeführten Merkmale. Das Verfahren ist geeignet zur Herstellung und Reinigung von α-Interferonen aus verschiedenen Spezies, wie z.B. humanen oder tierischen α-Interferonen. Der zur Herstellung verwendete Wirtsorganismus kann ein Prokaryont oder Eukaryont, beispielsweise E. coli oder Saccharomyces cerevisiae, vorzugsweise E. coli sein. Die Kultivierungsbedingungen für die verschiedenen Wirtsorganismen sind dem Fachmann bestens bekannt.

Überraschenderweise wurde gefunden, daß die Wachstumszeit nicht nur Einfluß auf die Ausbeute an α-Interferon hat, sondern vor allem auch entscheidend mit dafür verantwortlich ist, wie das Interferongemisch zusammengesetzt ist. So änderte sich beispielsweise die Zusammensetzung des Interferongemisches bei Verwendung des E. coli hinsichtlich der Menge an Methionin-Interferon je nach Dauer des Wachstums.

Vorteilhafterweise wird daher der Fermentationsansatz in kurzen Zeitabständen auf die Bildung von durch den Wirtsorganismus hervorgerufenen α-Interferonderivaten, als Indikator für die beste Wachstumszeit, überprüft. Methionin-Interferon kann als ein solcher Indikator dienen. Durch zeitgerechten Abbruch beispielsweise nach der Bildung von weniger als 20 %, vorzugsweise von weniger als 5 % besonders bevorzugt von weniger als 1 % Methionin-Interferon, erhält man daher bereits ein besonders reines Interferon mit idealen Voraussetzungen für das nachfolgende erfindungsgemäße Reinigungsverfahren.

Besonders geeignet ist das Verfahren zur Herstellung von säurestabilem α-Interferon. Hierbei kann beispielsweise das Verfahren gemäß DE 34 32 196.9, bei dem die Zellen bei pH 2 in einem Homogenisator zerstört werden, eingesetzt werden.

Durch Einsatz einer Tandem Chromatographie, d.h. mit hintereinandergeschalteten chromatographischen Schritten an verschiedenen Adsorptionsmitteln mit geeigneten Wasch- und Elutionslösungen kann überraschenderweise ein Großteil der Verunreinigungen abgetrennt werden. Vorzugsweise wird hierbei eine Kombination aus einer Zellulose-Vorsäule direkt verbunden mit einer Affinitätssäule verwendet. Besonders bevorzugt ist eine DE-52-Zellulose mit einem an einem Träger, wie etwa Sepharose gekuppelten monoklonalen Anti-Interferon IgG-Antikörper, wie beispielsweise dem in der DEOS 33 06 060 beschriebenen EBI 1 Antikörper.

Als geeignete Waschlösung hat sich beispielsweise ein TRIS/NaCL-Puffer pH 7,5 ergeben, doch sind auch Waschlösungen geeignet, die die Bindung des Interferons an den Antikörper nicht beeinflussen, die Verunreinigungen jedoch herauswaschen, und die Bestandteile, die die Eigenschaften der Antikörpersäule negativ beeinflussen, an der Vorsäule gebunden lassen.

Ein geeignetes Elutionsmittel für das Interferon ist beispielweise eine Pufferlösung aus 0,1 M Zitronensäure in 25 %igem Ethylenglykol, jedoch sind auch andere Elutionsmittel geeignet, die ähnliche Eigenschaften besitzen. Generell muß das Elutionsmittel auf das jeweilige zu reinigende α-Interferon abgestimmt sein.

Ein Teil der Verunreinigungen des "Tandem-Eluates" ließ sich überraschenderweise bereits durch Abpufferung des pH-Wertes vorzugsweise auf pH 4,0 - 4,8, besonders bevorzugt auf pH 4,5 abtrennen. Der pH Wert sollte so gewählt werden, daß sich im Niederschlag möglichst wenig monomeres α-Interferon findet.

Die Endreinigung des Interferons ließ sich durch die Chromatographie an einem Kationenaustauscher, vorzugsweise mit einem MONO-S, Typ HR 10/10 Kationenaustauscher erreichen. Zur Elution des hochgereinigten α-Interferons wurde ein flacher Stufengradient mit einem flüchtigen Puffer, beispielsweise einem Ammoniumazetatpuffer verwendet, bei dem der pH-Wert konstant war und die Konzentration variiert wurde (Konzentrationsgradient). Ebensogut ließe sich auch die Konzentration konstant halten und der pH-Wert variieren (pH-Gradient). Entscheidend ist, daß das Elutionsmittel in der Lage ist, Interferonbeimengungen, insbesondere S-S-Isomere des hauptsächlich auftretenden Monomeren abzutrennen. Besonders geeignet ist hierzu ein flacher Konzentrationsgradient eines Ammoniumazetatpuffers, hergestellt aus 0,1 - 1,0 M, vorzugsweise aus 0,1 - 0,5 M Ammoniumazetat in einem pH-Bereich von 4,0 - 5,0, vorzugsweise bei pH 4,5. Dieser Puffer kann darüberhinaus durch Lyophilisation entfernt werden, so daß die hochgereinigten α-Interferone erstmals in fester Form, frei von Puffersalzen und Fällungsmitteln erhalten werden können.

Besonders geeignet ist das erfindungsgemäße Verfahren zur Herstellung von α-Interferonen verschiedener Spezies, die nach Applikation in den entsprechenden Spezies keine Antikörper stimulieren.

Als besonders vorteilhaft hat sich das erfindungsgemäße Verfahren bei der Herstellung eines rekombinanten, homogenen, reinen festen und nichtimmunogenen α-Interferons erwiesen, das frei von reduzierten Formen und Bruchstücken des Interferons ist, das weniger als 0,2 % Oligomere und weniger als 2 % Dimere/Trimere/Tetramere und weniger als 5 % Methionin-Interferon aufweist und bei dem mehr als 90 % des Monomerengehaltes aus nativ-monomerem α-Interferon bestehen.

Als besonders vorteilhaft läßt sich das erfindungsgemäße Verfahren bei der Herstellung eines wie oben beschriebenen α-Interferons einsetzen, bei dem der Wirtsorganismus das für das Human-α-Interferon gemäß der Aminosäuresequenz

```
Cys Asp Leu Pro Gln Thr His Ser Leu Gly Ser Arg Arg Thr Leu
Met Leu Leu Ala Gln Met Arg Arg Ile Ser Leu Phe Ser Cys Leu
Lys Asp Arg Arg Asp Phe Gly Phe Pro Gln Glu Glu Phe Gly Asn
Gln Phe Gln Lys Ala Glu Thr Ile Pro Val Leu His Glu Met Ile
Gln Gln Ile Phe Asn Leu Phe Ser Thr Lys Asp Ser Ser Ala Ala
Trp Asp Glu Thr Leu Leu Asp Lys Phe Tyr Thr Glu Leu Tyr Gln
Gln Leu Asn Asp Leu Glu Ala Cys Val Ile Gln Gly Val Gly Val
Thr Glu Thr Pro Leu Met Lys Glu Asp Ser Ile Leu Ala Val Arg
Lys Tyr Phe Gln Arg Ile Thr Leu Tyr Leu Lys Glu Lys Lys Tyr
Ser Pro Cys Ala Trp Glu Val Val Arg Ala Glu Ile Met Arg Ser
Phe Ser Leu Ser Thr Asn Leu Gln Glu Ser Leu Arg Ser Lys Glu
```

kodierende Gen enthält und das nativ monomere α-Interferon je eine Disulfidbrücke zwischen den Cysteinen an Position 1 und 98 und 29 und 138 aufweist.

Gegenstand der vorliegenden Erfindung sind auch rekombinante α-Interferone, die nach dem erfindungsgemäßen Verfahren herstellbar sind, vorzugsweise rekombinante α-Interferone in homogener, reiner Form, die weniger als 20 %, vorzugsweise weniger als 5 %, besonders bevorzugt weniger als 1 % Methionin-Interferon enthalten.

Bedingt durch die Wahl des Wirtsorganismus und die Art der Fermentationsbedingungen können rekombinante hergestellte α-Interferone neben dem Methionin-Interferon auch Di-, Tri-, Tetra-und Oligomere,reduzierte Formen und Bruchstücke und S-S-Isomere des hauptsächlich auftretenden monomeren α-Interferons in wechselnden Mengen enthalten, die, wie beschrie ben, nach dem erfindungsgemäßen Verfahren erstmals unterdrückt, beziehungsweise beseitigt werden können.

Ein weiterer Gegenstand der Erfindung sind daher rekombinante α-Interferone in homogener, reiner Form, die weniger als 20 %, vorzugsweise weniger als 5 %, besonders bevorzugt weniger als 1 % Methionin-Interferon enthalten, die frei von reduzierten Formen und Bruckstücken des α-Interferons sind, die weniger als 0,2 % Oligomere und weniger als 2 % Dimere/Trimere/Tetramere, vorzugsweise keine Oligo-, Tetra-, Tri- und Dimere enthalten, die bevorzugt mehr als 90 % natives Monomeres enthalten, und vorzugsweise völlig frei von S-S-Isomeren des nativ-monomeren α-Interferons sind.

Gegenstand der Erfindung sind weiterhin die beschriebenen rekombinanten α-Interferone verschiedener Spezies, die nach Applikation in jeweiligen Spezies keine Antikörper stimulieren, ebenso wie auch feste Interferone.

Bevorzugt ist ein rekombinantes Human-α-Interferon mit den oben beschriebenen Eigenschaften, vorzugsweise ein rekombinantes Human-α-Interferon gemäß der Aminosäuresequenz

```
Cys Asp Leu Pro Gln Thr His Ser Leu Gly Ser Arg Arg Thr Leu
Met Leu Leu Ala Gln Met Arg Arg Ile Ser Leu Phe Ser Cys Leu
Lys Asp Arg Arg Asp Phe Gly Phe Pro Gln Glu Glu Phe Gly Asn
Gln Phe Gln Lys Ala Glu Thr Ile Pro Val Leu His Glu Met Ile
Gln Gln Ile Phe Asn Leu Phe Ser Thr Lys Asp Ser Ser Ala Ala
Trp Asp Glu Thr Leu Leu Asp Lys Phe Tyr Thr Glu Leu Tyr Gln
Gln Leu Asn Asp Leu Glu Ala Cys Val Ile Gln Gly Val Gly Val
Thr Glu Thr Pro Leu Met Lys Glu Asp Ser Ile Leu Ala Val Arg
Lys Tyr Phe Gln Arg Ile Thr Leu Tyr Leu Lys Glu Lys Lys Tyr
Ser Pro Cys Ala Trp Glu Val Val Arg Ala Glu Ile Met Arg Ser
Phe Ser Leu Ser Thr Asn Leu Gln Glu Ser Leu Arg Ser Lys Glu.
```

Besonders bevorzugt ist ein rekombinantes, nichtimmunogenes, festes Human-α-Interferon, das der Aminosäuresequenz

```
Cys Asp Leu Pro Gln Thr His Ser Leu Gly Ser Arg Arg Thr Leu
Met Leu Leu Ala Gln Met Arg Arg Ile Ser Leu Phe Ser Cys Leu
Lys Asp Arg Arg Asp Phe Gly Phe Pro Gln Glu Glu Phe Gly Asn
Gln Phe Gln Lys Ala Glu Thr Ile Pro Val Leu His Glu Met Ile
Gln Gln Ile Phe Asn Leu Phe Ser Thr Lys Asp Ser Ser Ala Ala
Trp Asp Glu Thr Leu Leu Asp Lys Phe Tyr Thr Glu Leu Tyr Gln
Gln Leu Asn Asp Leu Glu Ala Cys Val Ile Gln Gly Val Gly Val
Thr Glu Thr Pro Leu Met Lys Glu Asp Ser Ile Leu Ala Val Arg
Lys Tyr Phe Gln Arg Ile Thr Leu Tyr Leu Lys Glu Lys Lys Tyr
Ser Pro Cys Ala Trp Glu Val Val Arg Ala Glu Ile Met Arg Ser
Phe Ser Leu Ser Thr Asn Leu Gln Glu Ser Leu Arg Ser Lys Glu.
```

entspricht und in homogener, reiner Form vorliegt, mit weniger als 5 % Methionin-Interferon,
das frei von reduzierten Formen und Bruckstücken des Interferons ist,
mit weniger als 0,2 % Oligomeren und weniger als 2 % Dimeren/Trimeren/Tetrameren und
bei dem mehr als 95 % des Monomerengehaltes aus dem nativ-monomeren α-Interferon mit je einer Disulfidbrücke zwischen den Cysteinen an Position 1 und 98 und 29 und 138 besteht.

Das erfindungsgemäße Verfahren ermöglicht die Abtrennung der Verunreinigungen und der Interferon-Beimengungen unter schonendsten Bedingungen. Es soll am Beispiel des Interferons-$\alpha_2$Arg gemäß der Aminosäuresequenz

```
Cys Asp Leu Pro Gln Thr His Ser Leu Gly Ser Arg Arg Thr Leu
Met Leu Leu Ala Gln Met Arg Arg Ile Ser Leu Phe Ser Cys Leu
Lys Asp Arg Arg Asp Phe Gly Phe Pro Gln Glu Glu Phe Gly Asn
Gln Phe Gln Lys Ala Glu Thr Ile Pro Val Leu His Glu Met Ile
Gln Gln Ile Phe Asn Leu Phe Ser Thr Lys Asp Ser Ser Ala Ala
Trp Asp Glu Thr Leu Leu Asp Lys Phe Tyr Thr Glu Leu Tyr Gln
Gln Leu Asn Asp Leu Glu Ala Cys Val Ile Gln Gly Val Gly Val
Thr Glu Thr Pro Leu Met Lys Glu Asp Ser Ile Leu Ala Val Arg
Lys Tyr Phe Gln Arg Ile Thr Leu Tyr Leu Lys Glu Lys Lys Tyr
Ser Pro Cys Ala Trp Glu Val Val Arg Ala Glu Ile Met Arg Ser
Phe Ser Leu Ser Thr Asn Leu Gln Glu Ser Leu Arg Ser Lys Glu
```

näher erläutert werden, doch sind auch andere α-Interferone,wenn erforderlich mit geringen nicht erfinderi schen Modifikationen,nach dem erfindungsgemäßen Verfahren herzustellen und zu reinigen.

Die säure-gefällte tiefgefrorene Biomasse wurde durch Rühren in 1 %iger Essigsäure aufgetaut. Diese und alle nachfolgenden Manipulationen wurden bei etwa 5°C durchgeführt.

Die Extraktion des Proteins aus den Zellen erfolgte, wie in der Patentanmeldung DE 34 32 196.9 ausführlich beschrieben, durch Aufbrechen der Bakterienzellen in einem Homogenisator, Zugabe eines Fällungshilfsmittels wie beispielweise Polyethylenimin PEI-600 in einem Konzentrationsbereich von 0,1 - 0,25 %, Einstellung des pH-Wertes mit Hilfe von NaOH auf 7,5 - 10,0 und Rühren der Suspension für mehrere Stunden. Anschließend wurde der pH-Wert auf 7,5 eingestellt, der Rohextrakt auf schonende Weise geklärt und Proben für die Proteinbestimmung und den Interferon-Test entnommen.

Trotz der bekannten Empfindlichkeit der Polypeptide, wie Interferon gegenüber den bei mechanischen Einflüssen auftretenden Scherkräften (Proc. Soc. Exp. Biol. Med. 146, 249 - 253 (1974)) konnten durch dieses Verfahren unter diesen pH-Bedingungen überraschend hohe Ausbeuten an Rohinterferon erzielt werden.

Die Überprüfung verschiedener Fermentationsansätze zeigte, daß sich die Zusammensetzung der Mischung abhängig von den Fermentationsbedingungen änderte.

Insbesondere der Anteil an der Komponente 1, das Methionin-Interferon-α., eine kaum abtrennbare Komponente, änderte sich mit der Dauer der Fermentation: Methionin-Interferon-α wurde erst nach 8 - 9 Stunden Fermentationsdauer gebildet.

Durch zeitgerechten Abbruch der Fermentation lassen sich also Interferonpräparationen erhalten, die kein Methionin-Interferon enthalten.

In die geklärte Rohlösung wurde unter Rühren festes Ammonsulfat bis zu 65 % Sättigung zugegeben. Nach vollständiger Lösung des Ammonsulfates wurde der Ansatz gekühlt über Nacht gehalten, der entstandene Niederschlag abgetrennt und bis zur weiteren Verwendung bei -20°C aufbewahrt.

Aus dem klaren Überstand wurden wiederum Proben für den Interferon-Test entnommen, um die Fällung des Interferons zu kontrollieren. Es sollten nicht mehr als 5 % des Interferons im Überstand verbleiben.

Das Ammonsulfat-Pellet wurde in 0,01 M NaCl gelöst, der pH-Wert mit NaOH auf 7,5 eingestellt und die Lösung 2 Stunden gerührt. Der unlösliche Anteile wurde abgetrennt und eventuell nochmals mit 0,01 M NaCl extrahiert.

Die vereinigten klaren Lösungen wurden mit Hilfe einer sterilen, pyrogenfreien Dialysierpatrone gegen 0,01 M NaCl dialysiert. Die Osmolarität der Interferon-Lösung sollte nach 2-3-maligem Durchsatz etwa 390 - 430 mOsmol/l betragen. Aus der geklärten Lösung wurden Aliquote für den Interferon-Test entnommen.

Zur weiteren Reinigung wurde die "Tandem-Chromatographie" eingesetzt; eine Kombination aus einer Zellulose-Vorsäule und einer nachgeschalteten Affinitätschromatographie mit hochspezifischen, monoklonalen Antikörpern. Die Vorsäule, eine Ionenaustauscher-Säule, diente dazu, schwerlösliche Probenbestandteile von der Antikörpersäule fernzuhalten.

Für die Vorsäule wurde DE-52-Zellulose (Fa. Whatman) mit TRIS/NaCl-Puffer, pH 7,5, gut verrührt und in eine Chromatographie-Säule gefüllt. Das Adsorbens wurde mit dem Puffer so lange gewaschen, bis das Eluat keine pH- und Osmolaritätsänderungen mehr zeigte. Für die Vorsäule wurde 0,5 - 1,0 g DE 52-Zellulose in 0,025 M TRIS/HCl + 0,2 M NaCl pro Gramm Biomasse verwendet; sie wurde für jede Reinigung neu bereitet.

Für die Antikörpersäule wurde aus Mäuseascites gewonnenes und gereinigtes monoklonales Anti-Interferon-IgG an BrCN-aktivierte Sepharose 4B (Fa. Pharmacia) als Träger gekuppelt. Das fertige Säulenmaterial wurde in phosphatgepufferter Kochsalzlösung (PBS) mit Natriumazid im Kühlraum aufbewahrt. Vor dem erstmaligen Gebrauch oder nach längerer Aufbewahrung wurde die Antikörper-Säule mit 0,1 M Zitronensäure in 25 % Ethylenglykol gewaschen, um etwaige lösliche Anteile zu entfernen, und anschließend mit PBS neutral gewaschen. Pro Gramm Biomasse wurde bei der Antikörpersäule ein Säulenvolumen von 0,2 - 1,0 ml benötigt; diese Säule konnte mehrfach verwendet werden. Die dialysierte Interferonlösung wurde zunächst über beide Säulen (Vorsäule und Antikörpersäule) gepumpt und das Eluat durch Messung der Extinktion bei 280 nm kontrolliert. Nach dem Auftragen der Interferonlösung wurde solange mit TRIS/NaCl-Puffer pH 7,5 gewaschen, bis die Proteinmenge im Eluat auf 1/20 des Plateau-Wertes abgefallen war. Um zu kontrollieren, daß das Interferon an die Antikörpersäule gebunden worden war, wurde das Eluat auf Interferongehalt getestet. Die Antikörpersäule wurde anschließend von der Vorsäule getrennt und allein mit TRIS/NaCl-Puffer pH 7,5 gewaschen, bis im Eluat kein Protein mehr nachweisbar war.

Die Elution des an den Antikörper gebundenen Interferons erfolgte mit 0,1 M Zitronensäure in 25 % Ethylenglykol, wobei die Extinktion des Eluates bei 280 nm verfolgt wurde. Der das Interferon enthaltende Protein-Peak wurde gesammelt. Bis zur Endreinigung wurde der Interferon-Pool bei -20°C aufbewahrt. Interferontest, Proteinbestimmung und die Reverse-Phase HPLC-Analyse zeigten, daß durch diese Reinigung 60 - 90 % reines IFN-α erhalten wurde. Neben oligomeren Formen enthielt dieser Interferon-Pool reduzierte Formen mit freien SH-Gruppen, Dimere, Trimere, Tetramere und das nicht-native Monomere. Diese Komponenten sind alle biologisch und immunologisch als IFN charakterisierbar. Überraschenderweise ließ sich ein Teil dieser Komponenten durch die Ausfällung bei pH 4,5 (mit Ammoniak) entfernen. Hierbei wurden insbesondere die Anteileder Komponenten mit reduzierten Schwefelbrücken, also die Formen mit freien SH-Gruppen (Komponente 5 und 6) reduziert. Die Analyse dieses Niederschlages zeigt, daß das monomere Interferon nur in geringen Mengen mitgerissen wurde.

Zur Endreinigung wurde eine FPLC-Apparatur der Firma Pharmacia mit einer MONO-S-Säule, Type HR 10/10 (Kationenaustauscher) verwendet, die mit bis zu 60 mg Protein beladen werden konnte. Dieses Säulenmaterial stellt einen Hochleistungsionenaustauscher dar,mit hervorragender Trennwirkung und dem großen Vorteil, daß trotz der relativ großen Menge an zu reinigendem Protein die Endreinigung nur wenige Stunden dauerte, die Pufferlösungen sterilfiltriert eingesetzt werden konnten und bei Raumtemperatur gearbeitet werden konnte.

Der nach der Fällung erhaltene klare Überstand wurde auf die Säule aufgetragen. Von besonderer Bedeutung war der bei der FPLC-Trennung verwendete Puffer. Er mußte die Interferonkomponenten deutlich unterscheidbar eluieren und nachher vollständig entfernbar sein. Diese Eigenschaften besaß der verwendete Ammonium-Acetat-Puffer mit dem das Interferon durch ein Gradientenprogramm eluiert wurde. Interferon wurde als scharfer Peak mit einer schwachen Schulter eluiert. Es wurde sowohl die "Schulter"-Fraktion (K 3), als auch die später eluierten Anteile (K 5 - K 7) von dem Hauptpeak des reinen Interferons abgetrennt. Der Peak des reinen Interferons wurde gesammelt und aus diesem Aliquote für die HPLC-Analyse, SDS-Gelelektrophorese, Proteinbestimmung, Interferon-Test und Endotoxinbestimmung entnommen.

Durch diese Chromatographie wurden praktisch alle Komponenten vom Hauptpeak abgetrennt und ein homogenes Interferon erhalten, das bei der Gelpermeations-HPLC einen Monomerengehalt von über 99 % zeigte. Die Reverse-Phae HPLC zeigte nur mehr ca. 1 % an dem nicht-nativen Monomer, die Chromatofokussierung einen Anteil von 2,5 % nicht-natives Monomer.

Die MONO-S-Säule wurde der Wiederverwendung mit 0,5 M NACl + 0,1 M Na-Phosphat, pH 8,0 gewaschen, um adsorbierte Verunreinigungen zu entfernen; aufbewahrt wurde sie in 25 % Ethanol.

Der flüchtige Puffer konnte durch Lyophilisation vollständig entfernt werden. Dazu wurde der IFN-Pool in Portionen bis zu maximal je 2 ml in au toklavierte Lyo-Ampullen (Volumen 8 ml) abgefüllt; das entsprach pro Ampulle einer Menge von 1 bis zu etwa 8 mg reinem Interferon. Die Ampullen wurden sodann mit vorgewaschenen und autoklavierten Lyo-Stopfen versehen und auf mindestens -20°C gekühlt. Die Lyophilisation erfolgte bei -10°C und einem Vakuum von unter 1 Torr. Nach Entfernung der Pufferlösung wurde die Temperatur auf 25°C erhöht und mindestens 1 Stunde lang weiterlyophilisiert. Das Vakuum wurde aufgehoben und die Stopfen sofort fest angedrückt. Versehen mit Alu-Verschlüssen wurden die Ampullen anschließend im Kühlraum oder bei -20°C gelagert.

Wie gezeigt wurde, ist des durch eine geschickte Fermentationsführung (relativ frühzeitige Ernte) zusammen mit dem erfindungsgemäßen Verfahren erstmals möglich geworden, ein Interferon-α herzustellen, das nicht nur hinsichtlich seines Interferon-Gehaltes bei über 98 % Reinheit liegt, sondern auch hinsichtlich der Homogenität bezogen auf die verschiedenen Interferonkomponenten zu mehr als 95 % aus nativem monomeren Interferon-α besteht.

Dieser hohe Reinheits- und Homogenitätsgrad ermöglichte darüberhinaus auch, das Interferon erstmals, frei von Salzen und Pufferbestandteilen, in fester Form zu erhalten. Es ist daher erstmals möglich, α-Interferon über Monate ohne Stabilisatoren zu lagern, was für die Lagerung, den Versand und nicht zuletzt für die galenischen Entwicklungen bedeutende Vorteile gegenüber den bisher stets mit Albumin stabilisierten Interferonen bietet. Auch nach 11 Monaten Lagerung bei 4°C konnte kein Gehaltsverlust festgestellt werden.

Bei dem in der EPA 83 734 beschriebenen kristallinen Human-Leukozyten-Interferon handelt es sich um Kristalle des Polyethylenglykol als Fällungsmittel mit Interferon, jedoch nicht um reines, homogenes und kristallines Interferon wie der Titel glauben machen möchte.

Das erfindungsgemäß hergestellte Interferon-α wurde wie bekannt durch Zugabe von menschlichem Serum Albumin gelöst, steril filtriert und aseptisch auf Fläschchen verteilt; je nach Applikation in geeigneten Konzentrationen.

Bei den klinischen Untersuchungen erwies sich das erfindungsgemäß hergestellte Interferon-α als nichtimmunogen und hervorragend verträglich.

Insgesamt wurden bis Januar 1985 75 Patienten mit dem nichtimmunogenen Interferon-α behandelt: 58 Patienten mit Tumorindikationen und 17 mit viralen Indikationen.

Bei keinem einzigen Patienten wurden während der Therapie Antikörper stimuliert, wobei die Behandlungsdauer teilweise 15 und mehr, bis zu 35 Wochen ausmachte.

Das erfindungsgemäße Verfahren ermöglichte es erstmals ein hochreines, bezogen auf das native, monomere Interferon homogenes, festes, frei von Salzen und Pufferbestandteilen und nichtimmunogenes Interferon-$\alpha_2$ bereitzustellen.

Die Aminosäuresequenzanalyse nach bekannten Verfahren ergab die nachfolgende Aminosäuresequenz:

```
Cys Asp Leu Pro Gln Thr His Ser Leu Gly Ser Arg Arg Thr Leu
Met Leu Leu Ala Gln Met Arg Arg Ile Ser Leu Phe Ser Cys Leu
Lys Asp Arg Arg Asp Phe Gly Phe Pro Gln Glu Glu Phe Gly Asn
Gln Phe Gln Lys Ala Glu Thr Ile Pro Val Leu His Glu Met Ile
Gln Gln Ile Phe Asn Leu Phe Ser Thr Lys Asp Ser Ser Ala Ala
Trp Asp Glu Thr Leu Leu Asp Lys Phe Tyr Thr Glu Leu Tyr Gln
Gln Leu Asn Asp Leu Glu Ala Cys Val Ile Gln Gly Val Gly Val
Thr Glu Thr Pro Leu Met Lys Glu Asp Ser Ile Leu Ala Val Arg
Lys Tyr Phe Gln Arg Ile Thr Leu Tyr Leu Lys Glu Lys Lys Tyr
Ser Pro Cys Ala Trp Glu Val Val Arg Ala Glu Ile Met Arg Ser
Phe Ser Leu Ser Thr Asn Leu Gln Glu Ser Leu Arg Ser Lys Glu
```

Die folgenden Beispiele sollen die Erfindung näher erläutern, ohne sie in irgendeiner Form einzuschränken.

Vor allem hinsichtlich des Fermentationsansatzes ist das erfindungsgemäße Verfahren in weiten Grenzen uneingeschränkt einsetzbar. So ist es ebenfalls möglich, Biomasse anderer Wirtsorganismen zu verwenden, die nach dem mechanischen Aufschluß vergleichbare IFN-Ausbeuten erbringen und bei Interferonen, die ähnlich spezifisch mit dem EBI-1 monoklonalen Antikörper reagieren, Z.B. IFN-$\alpha_1$. Auch andere Interferone mit geringerer Homologie zum Interferon-alpha lassen sich bei Verwendung eines entsprechenden hochspezifischen monoklonalen Antikörpers nach dem erfindungsgemäßen Verfahren reinigen.

Gegenstand der vorliegenden Erfindung sind im Einzelnen Verfahren zur Herstellung von rekombinantem alpha-Interferon: dadurch gekennzeichnet, daß

der das Interferongen enthaltende Wirtsorganismus unter üblichen Bedingungen kultiviert wird,

die Zellen nach üblicher Wachstumszeit abgetötet und geerntet werden,

das exprimierte Interferon in üblicher Weise abgetrennt wird,

Zelltrümmer im schwach alkalischen abgetrennt werden,

das Interferon konzentriert und durch eine Tandem-Chromatographie vorgereinigt wird,

das Eluat zur Abtrennung von Verunreinigungen auf pH 4,0-4,8 eingestellt wird,

das Interferon durch Chromatographie an einer Kationenaustauschersäule mit einem flüchtigen Puffer als Elutionsmittel endgereinigt und anschließend lyophilisiert wird;

dadurch gekennzeichnet, daß der Wirtsorganismus E. coli ist;

dadurch gekennzeichnet, daß die Zellen nach einer Wachstumszeit, in der nicht mehr als 20%, vorzugsweise nicht mehr als 5% besonders bevorzugt nicht mehr als 1% Methionin-Interferon gebildet wird, abgetötet werden;

dadurch gekennzeichnet, daß die Zellen in einem Homogenisator bei pH 2 zerstört werden und das Interferon abgetrennt wird;

dadurch gekennzeichnet, daß die Tandem-Chromatographie aus einer Zellulose-Vorsäule und einer Affinitätschromatographie besteht und daß die zu reinigende Substanz über beide Säulen mit einer geeigneten Waschlösung gewaschen und das α-Interferon anschließend mit einem geeigneten Elutionsmittel von der Affinitätssäule eluiert wird;

dadurch gekennzeichnet, daß die Vorsäule mit DE-52 Zellulose, die Affinitätssäule mit einem an einem Träger gekuppelten monoklonalen Anti-Interferon IgG Antikörper beschickt wird und daß die zu reinigende Substanz mit einem TRIS-NaCl-Puffer, pH 7,5 über beide Säulen gewaschen und das α-Interferon anschließend mit 0,1 M Zitronensäure in 25 %igem Ethylenglykol von der Affinitätssäule eluiert wird,

dadurch gekennzeichnet, daß der monoklonale Antikörper EBI 1 verwendet wird;

dadurch gekennzeichnet, daß das Eluat der Tandem-Chromatographie zur Abtrennung von Verunreinigungen auf pH 4,5 eingestellt wird;

dadurch gekennzeichnet, daß zur Endreinigung ein MONO-S, Typ HR 10/10 Kationenaustauscher verwendet wird;

dadurch gekennzeichnet, daß zur Elution ein flacher Konzentrationsgradient, hergestellt aus 0,1 - 1,0 M, vorzugsweise aus 0,1 - 0,5 M Ammoniumazetat-Puffer bei pH-Werten von 4,0 - 5,0 vorzugsweise bei pH 4,5 eingesetzt wird.

Verfahren zur Herstellung eines festen α-Interferons.

Verfahren zur Herstellung eines nicht immunogenen α-Interferons.

Verfahren zur Herstellung eines rekombinanten, homogenen, reinen α-Interferons, das weniger als 5% Methionin-Interferon aufweist, das frei von reduzierten Formen und Bruchstücken des Interferons ist, das weniger als 0,2% Oligomere und weniger als 2% Dimere/Trimere/Tetramere enthält und bei dem mehr als 90% des Monomerengehaltes aus nativ-monomerem Interferon besteht.

Verfahren, dadurch gekennzeichnet, daß der Wirtsorganismus das für das Human-α-Interferon der Aminosäuresequenz

```
Cys Asp Leu Pro Gln Thr His Ser Leu Gly Ser Arg Arg Thr Leu
Met Leu Leu Ala Gln Met Arg Arg Ile Ser Leu Phe Ser Cys Leu
Lys Asp Arg Arg Asp Phe Gly Phe Pro Gln Glu Glu Phe Gly Asn
Gln Phe Gln Lys Ala Glu Thr Ile Pro Val Leu His Glu Met Ile
Gln Gln Ile Phe Asn Leu Phe Ser Thr Lys Asp Ser Ser Ala Ala
Trp Asp Glu Thr Leu Leu Asp Lys Phe Tyr Thr Glu Leu Tyr Gln
Gln Leu Asn Asp Leu Glu Ala Cys Val Ile Gln Gly Val Gly Val
Thr Glu Thr Pro Leu Met Lys Glu Asp Ser Ile Leu Ala Val Arg
Lys Tyr Phe Gln Arg Ile Thr Leu Tyr Leu Lys Glu Lys Lys Tyr
Ser Pro Cys Ala Trp Glu Val Val Arg Ala Glu Ile Met Arg Ser
Phe Ser Leu Ser Thr Asn Leu Gln Glu Ser Leu Arg Ser Lys Glu.
```

kodierende Gen enthält.

Verfahren zur Herstellung eines rekombinanten, homogenen, reinen, Human-α-Interferons der Aminosäuresequenz

```
Cys Asp Leu Pro Gln Thr His Ser Leu Gly Ser Arg Arg Thr Leu
Met Leu Leu Ala Gln Met Arg Arg Ile Ser Leu Phe Ser Cys Leu
Lys Asp Arg Arg Asp Phe Gly Phe Pro Gln Glu Glu Phe Gly Asn
Gln Phe Gln Lys Ala Glu Thr Ile Pro Val Leu His Glu Met Ile
Gln Gln Ile Phe Asn Leu Phe Ser Thr Lys Asp Ser Ser Ala Ala
Trp Asp Glu Thr Leu Leu Asp Lys Phe Tyr Thr Glu Leu Tyr Gln
Gln Leu Asn Asp Leu Glu Ala Cys Val Ile Gln Gly Val Gly Val
Thr Glu Thr Pro Leu Met Lys Glu Asp Ser Ile Leu Ala Val Arg
Lys Tyr Phe Gln Arg Ile Thr Leu Tyr Leu Lys Glu Lys Lys Tyr
Ser Pro Cys Ala Trp Glu Val Val Arg Ala Glu Ile Met Arg Ser
Phe Ser Leu Ser Thr Asn Leu Gln Glu Ser Leu Arg Ser Lys Glu
```

das weniger als 5% Methionin-Interferon aufweist,

das frei von reduzierten Formen und Bruchstücken des α-Interferons ist, das weniger als 0,2 % Oligomere und weniger als 2% Dimere/Trimere/Tetramere enthält und bei dem das nativ monomere α-Interferon je eine Disulfidbrücke zwischen den Cysteinen an Position 1 und 98 und 29 und 138 aufweist.

Weiterhin sind rekombinante α-Interfrone, herstellbar nach einem der erfindungsgemäßen Verfahren, Gegenstand der vorliegenden Erfindung:

dadurch gekennzeichnet, daß es in homogener, reiner Form, mit weniger als 20%, vorzugsweise weniger als 5% besonders bevorzugt weniger als 1% Methionin-Interferon vorliegt;

dadurch gekennzeichnet, daß es in homogener, reiner Form, frei von reduzierten Formen und Bruchstücken vorliegt und weniger als 0,2% Oligomere und weniger als 2% Dimere/Trimere/Tetramere enthält;

frei von Oligomeren und Dimeren/Trimeren/Tetrameren;

bei denen mehr als 90% des Monomerengehaltes nativ-monomeres α-Interferon ist;

frei von nicht-nativ-monomerem Interferon;

dadurch gekennzeichnet, daß es keine Antikörper stimuliert;

dadurch gekennzeichnet, daß es in fester Form vorliegt.

Humanes α-Interferon gemäß der Erfindung.

α-Interferon, dadurch gekennzeichnet, daß es der Aminosäuresequenz

```
Cys Asp Leu Pro Gln Thr His Ser Leu Gly Ser Arg Arg Thr Leu
Met Leu Leu Ala Gln Met Arg Arg Ile Ser Leu Phe Ser Cys Leu
Lys Asp Arg Arg Asp Phe Gly Phe Pro Gln Glu Glu Phe Gly Asn
Gln Phe Gln Lys Ala Glu Thr Ile Pro Val Leu His Glu Met Ile
Gln Gln Ile Phe Asn Leu Phe Ser Thr Lys Asp Ser Ser Ala Ala
Trp Asp Glu Thr Leu Leu Asp Lys Phe Tyr Thr Glu Leu Tyr Gln
Gln Leu Asn Asp Leu Glu Ala Cys Val Ile Gln Gly Val Gly Val
Thr Glu Thr Pro Leu Met Lys Glu Asp Ser Ile Leu Ala Val Arg
Lys Tyr Phe Gln Arg Ile Thr Leu Tyr Leu Lys Glu Lys Lys Tyr
Ser Pro Cys Ala Trp Glu Val Val Arg Ala Glu Ile Met Arg Ser
Phe Ser Leu Ser Thr Asn Leu Gln Glu Ser Leu Arg Ser Lys Glu.
```

entspricht.

Rekombinantes, Human-α-Interferon, das der Aminosäuresequenz

```
Cys Asp Leu Pro Gln Thr His Ser Leu Gly Ser Arg Arg Thr Leu
Met Leu Leu Ala Gln Met Arg Arg Ile Ser Leu Phe Ser Cys Leu
Lys Asp Arg Arg Asp Phe Gly Phe Pro Gln Glu Glu Phe Gly Asn
Gln Phe Gln Lys Ala Glu Thr Ile Pro Val Leu His Glu Met Ile
Gln Gln Ile Phe Asn Leu Phe Ser Thr Lys Asp Ser Ser Ala Ala
Trp Asp Glu Thr Leu Leu Asp Lys Phe Tyr Thr Glu Leu Tyr Gln
Gln Leu Asn Asp Leu Glu Ala Cys Val Ile Gln Gly Val Gly Val
Thr Glu Thr Pro Leu Met Lys Glu Asp Ser Ile Leu Ala Val Arg
Lys Tyr Phe Gln Arg Ile Thr Leu Tyr Leu Lys Glu Lys Lys Tyr
Ser Pro Cys Ala Trp Glu Val Val Arg Ala Glu Ile Met Arg Ser
Phe Ser Leu Ser Thr Asn Leu Gln Glu Ser Leu Arg Ser Lys Glu
```

entspricht, in homogener, reiner Form vorliegt, mit weniger als 5% Methionin-Interferon, weniger als 0,2% Oligomeren und weniger als 2% Dimeren/Trimeren/Tetrameren, das frei von reduzierten Formen und Bruchstücken des α-Interferons ist und bei dem mehr als 90% des Monomerengehaltes aus dem nativ-monomeren α-Interferon mit je einer Disulfidbrücke zwischen den Cysteinen an Position 1 und 98 und 29 und 138 besteht.

Verwendung eines α-Interferons gemäß der Erfindung zur therapeutischen Behandlung viraler und tumoraler Erkrankungen.

Pharmazeutisches Mittel zur therapeutischen Behandlung, dadurch gekennzeichnet, daß es ein α-Interferon gemäß der Erfindung und eine oder mehrere pharmazeutische, inerte Hilfs-und/oder Trägerstoffe enthält.

Beispiel 1 (Ansatz E.coli; IFN-$\alpha_2$ Arg; 28 °C)

a) 251 g säuregefällte Biomasse, die bei -20 °C gelagert worden war, wurde in 2500 ml 1%iger Essigsäure aufgenommen, 1/2 h im Eisbad gerührt und 2 mal 1 Minute mit Hilfe des Ultraturax Type 45/6 homogenisiert. Polymin P wurde bis zu einer Endkonzentration von 0,25 % zugegeben, der pH-Wert mit 5 N NaOH auf 10,0 eingestellt und 2 h im Eisbad gerührt, schließlich wurde der pH-Wert mit 5 N HCl auf 7,50 zurückgestellt.

1-Stündiges zentrifugieren in einer Christ Cryofuge 6 - 6 S bei 4 °C und 3000 Upm ergab einen klaren Rohextrakt von 2540 ml mit einem Interferongehalt von 17,1 x 10$^9$ I.E. ($\triangleq$100 %) und einem Proteingehalt von 5330 mg, woraus sich eine spezifische Aktivität von 3,21 x 10$^6$ I.E./mg Protein errechnete.

b) Ammoniumsulfat wurde bis zu 65 %iger Sättigung zugegeben (430 g / Liter Extrakt). Der Ansatz wurde über Nacht bei 4 - 8 °C aufbewahrt und der gebildete Niederschlag in einer Beckman J 2-21 Highspeed Zentrifuge, Rotor JA 10 bei 4 °C, 10.000 Upm innerhalb 1 Stunde abzentrifugiert. Der klare Überstand, 3120 ml, enthielt 0,7 % des im Rohextrakt enthaltenen Interferons (120 x 10$^6$ I.E.).

Das Pellet wurde in 0,01 M NaCl aufgenommen und bei 4 - 8 °C 2 h lang gerührt. Der pH-Wert wurde mit 5 N NaOH auf 7,50 eingestellt und die Lösung,wie oben beschrieben, klarzentrifugiert. Die klare Lösung wurde mit Hilfe einer Dialysierpatrone (Nephross Allegro, Fa. Organon Technika) gegen 0,01 M NaCl auf 390 mOsmol/l dialysiert.Der Interferongehalt betrug 13,3 x 10$^9$ I.E. ($\triangleq$ 77,6 %)

c) Das Dialysat wurde anschließend chromatographiert (Tandem-Chromatographie). Für die Vorsäule verwendete man 125 g DE 52-Zellulose Pulver der Firma Whatman in TRIS/NaCl-Puffer pH 7,5 (0,025 M TRIS/HCl + 0,2 M NaCl); das entsprach 0,5 g Säulenmaterial / g Biomasse. Für die Affinitätssäule wurde an Br-CN-aktivierte Sepharose 4 B (Firma Pharmacia) gekuppeltes monoklonales Anti-Interferon-IgG (EBI 1) verwendet. Das fertige Säulenmaterial wurde in phosphatgepufferter Kochsalzlösung (PBS) mit Natriumazid bei 4 - 8 °C aufbewahrt. Vor der Verwendung wurde die Antikörper-Säule mit 0,1 M Zitronensäure in 25 % Ethylenglykol gewaschen und anschließend mit PBS neutral gespült. Pro Gramm Biomasse wurde bei der Antikörpersäule ein Säulenvolumen von 0,2 - 1,0 ml benötigt. Die dialysierte Interferonlösung wurde zunächst über beide Säulen (Vorsäule und Antikörpersäule) gepumpt und das Eluat durch Messung der Extinktion bei 280 nm kontrolliert. Nach dem Auftragen der Interferonlösung wurde solange mit TRIS/NaCl-Puffer pH 7,5 gewaschen, bis die Proteinmenge im Eluat auf 1/20 des Plateau-Wertes abgefallen war. Die Antikörpersäule wurde anschließend von der Vorsäule getrennt und allein mit TRIS/NaCl-Puffer pH 7,5 gewaschen, bis im Eluat kein Protein mehr nachweisbar war.

Die Elution des an den Antikörper gebundenen Interferons erfolgte mit 0,1 M Zitronensäure in 25 % Ethylenglykol, wobei die Extinktion des Eluates wiederum bei 280 nm verfolgt wurde. Der das Interferon enthaltende Protein-Peak wurde gesammelt. Man erhielt 16,8 ml Eluat mit einem Interferongehalt von 12,3 x 10$^9$ I.E. (≙ 71,9 %). Die Gesamt-Proteinmenge betrug 54,4 mg, woraus sich eine spezifische Aktivität von 226 x 10$^6$ I.E./mg Protein errechnete.

d) Zur weiteren Reinigung wurde das Eluat mit Ammoniak auf pH 4,5 eingestellt und der gebildete Niederschlag abgetrennt. Der klare Überstand (18,3 ml) enthielt 46,3 mg Protein und einen Interferonanteil von 11,8 x 10$^9$ I.E. bezogen auf das Rohprotein. Das entsprach einer Ausbeute von 69 % (225 x 10$^6$ I.E./mg Protein)

e) Zur Endreinigung wurde ein FPLC-Apparatur der Firma Pharmacia mit einer MONO-S-Säule, Type HR 10/10 (Kationenaustauscher)verwendet.

Der nach der Fällung erhaltene klare Überstand wurde auf die Säule, die mit 0,1 M Ammonazetat-Puffer, pH 4,5 - 5,0 vorgewaschen worden war, aufgetragen und diese solange gewaschen, bis die Extinktion bei 280 nm auf den Ausgangswert zurückgegangen war. Die Elution des adsorbierten Interferons erfolgte mit einem flachen Salzgradienten durch Zumischen von 0,5 M Ammonazetatpuffer, pH 4,5 - 5,0. Interferon wurde als scharfer Peak eluiert. Es wurde sowohl die "Schulter"-Fraktion (K 3), als auch die später eluierten Anteile (K5 - K7) von dem Hauptpeak des reinen Interferon abgetrennt. Der Peak des reinen Interferons wurde gesammelt und aus diesem Aliquote für die HPLC-Analyse, SDA-Gelektrophorese, Proteinbestimmung, Interferon-Test und Endotoxinbestimmung entnommen. In der "Schulter"-Fraktion (9,1 ml) wurden insgesamt 4,1 mg Protein festgestellt; der Interferongehalt betrug 1,33 x 10$^9$I.E. (7,7 %). Daraus ergab sich 324 x 10$^6$ I.E./mg Protein.

Der Hauptpool von 9,8 ml enthielt 5,18 x 10$^9$I.E. (30,3 %) reinstes Interferon und insgesamt 16,1 mg Protein; daraus ergab sich eine spezifische Aktivität von 322 x 10$^6$ I.E./mg Protein.

f;

α) Der IFN-Pool wurde in Portionen bis zu maximal je 2 ml in autoklavierte Lyo-Ampullen (Volumen 8 ml) abgefüllt, das entsprach pro Ampulle einer Menge von 1 bis zu etwa 8 mg reinem Interferon. Die Ampullen wurden sodann mit vorgewaschenen und autoklavierten Lyo-Stopfen versehen und auf mindestens -20°C gekühlt. Die Lyophilisation erfolgte bei -10°C und einem Vakuum von unter 1 Torr. Nach Entfernung der Pufferlösung wurde die Temperatur auf 25°C erhöht und mindestens 1 Stunde lang weiterlyophilisiert. Das Vakuum wurde aufgehoben und die Stopfen sofort fest angedrückt. Versehen mit Alu-Verschlüssen wurden die Ampullen anschließend im Kühlraum oder bei -20°C gelagert.

β) Zur Überprüfung der Stabilität wurden vier verschiedene Fermentationsansätze, unabhängig von dem Beispiel 1a - f; α,jedoch analog gereinigt, lyophilisiert.

Die Lyophilisate wurden in IRMA-Verdünnungspuffer gelöst und mit Hilfe des NK2-IRMA für humanes IFN alpha (Fa. Celltech U.K.) analysiert.

| Charge | IFN-Titer vor Lyophilisation | IFN-Titer nach Lyophilisation | ± % |
|---|---|---|---|
| A | 720 x 10$^6$ | 754 x 10$^6$ | + 5 |
| B | 1337 x 10$^6$ | 1526 x 10$^6$ | + 14 |
| C | 981 x 10$^6$ | 852 x 10$^6$ | - 13 |
| D | 1230 x 10$^6$ | 1149 x 10$^6$ | - 7 |

Die Lyophilisation ergab also keine Verluste. Nach 11 Monaten Lagerung des Lyophilisates bei ca. 4°C (Kühlschrank) wurde es in 0,1 M Ammoniumacetat gelöst und sowohl auf Reinheit (durch Gelpermeations-HPLC) als auch auf Gehalt (durch den NK2-IRMA-Test) überprüft.

|  | vor der<br>Lyophilisation | nach 11 Monaten Lagerung<br>als Lyophilisat (4°C) |
|---|---|---|
| Reinheit<br>(Gel.-HPLC) | 98,5 % | 98,7 |
| IFN-Titer | $1510 \times 10^{6}$ Einh./ml | $1464 \times 10^{6}$ Einh./ml |

Beispiel 2

Zur Überprüfung des Einflusses der Fermentationszeit auf die Zusammensetzung der Interferonkomponenten wurden aus einem Fermentationsansatz (E. coli HB 101; 28°C) nach 8, 9, 10 oder 11 Stunden Proben entnommen, nach der üblichen Technik bei pH 2 mit Säure gefällt und nach der erfindungsgemäßen Methodik aufgearbeit und analysiert. Die folgende Tabelle zeigt den Gehalt der Proben an K 1, K 2 und K 3 (K 1: Met - IFN, K 2: natives IFN; K 3: nicht natives IFN). Die Werte wurden mit Hilfe der Chromatofokussierung ermittelt.

| Erntezeit nach Stunden | 8 | 9 | 10 | 11 |
|---|---|---|---|---|
| g feuchte Biomasse je Liter Kultur | 14 | 15 | 18 | 21 |
| mg IFN/g Biomasse (gemessen im Rohextrakt) | 0,28 | 0,21 | 0,16 | 0,12 |
| mg IFN/l Kulturvolumen | 3,9 | 3,2 | 2,9 | 2,5 |
| K 1 pI = 5,78 | 0,7 % | n.d.* | 10,7 % | 19,4 % |
| K 2 pI = 5,64 | 96,2 % | 97,5 % | 86,1 % | 78,5 % |
| K 3 pI = 5,49 | 3,1 % | 2,5 % | 3,3 % | 2,1 % |

* n.d. = not detected = nicht nachweisbar

Beispiel 3

Kupplung des EBI 1-Antikörper an CNBr-aktivierte Sepharose 4 B (vgl. DE-OS 33 06 060)

Der EBI-1 Antikörper wurde zunächst mit 0,5 M NaCl/0,2 M NaHCO$_3$, pH, 8,4 gelöst (in möglichst wenig Puffer) und gegen den Puffer so lange dialysiert, bis sich in der Außenlösung mit Bariumchlorid keine Sulfat-Ionen mehr nachweisen ließen. Eine sorgfältige Entfernung des Ammonsulfates war absolut erforderlich, da Ammonium-Ionen die nachfolgende Kupplung an den Träger stören. Die Proteinkonzentration wurde sodann auf 5 mg/ml mit Puffer e ingestellt. Zur Kupplung wirde als Träger CNBr-aktivierte Sepharose 4 B (Pharmacia) eingesetzt. Diese wurde nach Vorschrift des Herstellers (Beipackzettel) vorgewaschen. Für je 25 mg EBI-1-Antikörper wurde 1 g aktivierte Sepharose verwendet. Die Kupplung erfolgte in obigem Puffer, pH 8,4 bei Raumtemperatur 2 Stunden lang. Danach wurde die EBI-1-Sepharose nach Vorschrift des Beipackzettels abgenutscht und gewaschen. Im Filtrat durften nicht mehr als 5 % des eingesetzten EBI-1-Antikörpers verbleiben. Die fertige EBI-1-Sepharose wurde in PBS/Azid im Kühlraum aufbewahrt.

PBS/Azid:

PBS: 7,30 g Natriumchlorid p.A. (Merck 6404)
3,00 g Na$_2$HPO$_4$ x 2 H$_2$O p.A. (Merck 6580)
1,15 g NaH$_2$PO$_4$ x H$_2$O p.A. (Merck 6346) gelöst und auf 1000 ml aufgefüllt; pH. 7,0
Azid: Zu dem PBS wurden 1,0 g/l Natriumazid reinst (Merck 6688) zugegeben.

Die fertige Lösung wurde sterilfiltriert (0,2 mikron Porenweite) und im Kühlraum aufbewahrt.

Interferon Antikörper Assay (Neutralisationsassay)

Material

Zellen

Human Lungen Carcinoma Zellen "A-549" ATCC CCL 185.

Virus

Encephalomyocarditis Virus (EMC), ATCC VR 129.

Interferon Standard

HS-11 (1 Ampulle "HS-11" = lyophilisiertes Hu IFN r$\alpha$-A, aufgenommen in 1,2 ml H$_2$O, ergibt 12.000 iU/ml)

Gewebe Kultur Platten

96 Näpfe mit Deckel, flacher Boden, Corning, New York, No. 25860, Napf-Durchmesser 6,4 mm, Gewebe Kultur behandelt.

Medien

DMEM = Dulbecco's modifiziertes Eagle Medium, mit Glutamine, ohne Natriumbicarbonat, Flow Kat.No. 10-331-24 (1F-017D)
HEPES Sigma No. H-3375
TRICINE Calbiochem No. 33468, A grade
FCS = fötales Kalb Serum Boehringer Mannheim
HUMANSERUM-ALBUMIN Behring Inst., 20 %, zur Infusion Antiobioticum, Tiamulinhydrogenfumarat Biochemie Kundl/Tirol, Austria (Sandoz C.)
Wachstumsmedium: DMEM + 10 % FCS/inaktiviert 30 min/56 °C
+ 13 mM HEPES
+ 6 mM TRICINE
+ 1,6 g/l NaHCO$_3$
ohne Antibiotika
pH 7,2 - 7,4
Assay Medium: wie Wachstumsmedium, aber 5 % FCS anstatt 10 % und Zugabe 5 $\mu$g Tiamulin/ml
Verdünnungs-Medium:Wachstums Medium ohne Serum, mit 5$\mu$g/ml Tiamulin
Virus Medium: Wachstums Medium ohne Serum mit 5 $\mu$g/ml Tiamulin und mit 3,5 mg/ml Human Serum Albumin

```
Methylviolett-Vorratslösung:
Methylviolett Merck No. 1402 ..........   6 g
Ethanol      ........................... 100 ml
auflösen und bei ca. 50°C filtrieren

Methylviolett-Gebrauchslösung
Vorratslösung  ........................  50 ml
Wasser (pH neutral)  .................. 950 ml
```

Die Zellen wurden wie eine permanente Zellinie behandelt. Propagiert wurde durch Trypsinisation und Verdünnung im Wachstumsmedium. Für den Assay wurden die Zellen in einem Hemocytometer gezählt und in Assay Medium suspendiert, um eine Impflösung von 4 - 5 x 10⁴ Zellen pro ml pro Napf zu erhalten; diese wurde auf den Platten verteilt. Inkubiert wurde in einer Athmosphäre aus 5 % $CO_2$ und 80 % Luftfeuchtigkeit bei 37°C.

Nach 8 - 24 h war die Monoschicht üblicherweise vollständig. Zu diesem Zeitpunkt wurde Interferon und die Serumverdünnungen in getrennten Teströhrchen vorbereitet.

Für die Kontrollplatte wurden HS-11 Verdünnungen von 1 : 1000, 1 : 2000 ... bis 1 : 32.000 bei 37°C 1 h inkubiert.

Für die Probenplatte wurden die Serumproben auf 1 : 2, 1 : 4, 1: 8 ... bis 1 : 64 mit einem Verdünnungsmedium, das soviel HS-11 enthielt, um eine Endkonzentration von 10 iU HS-11/ml in jedem Glas zu erhalten, verdünnt und bei 37°C 1 h ebenso inkubiert.

Die Platten wurden dekantiert und jeder Napf wurde mit 100 ml des Verdünnungsmediums (Reihe 2, 3, 10 und 11) oder mit 100 $\mu$l der Verdünnungen (Reihe 4 - 9) gefüllt. Die Platten wurden bei 37°C 4 h wie oben inkubiert. Hiernach wurden die Platten mit 100 $\mu$l des Virus Mediums (ohne Virus)pro Napf und 50 $\mu$l der Virus Verdünnung (Reihe 3, 11, 4- 9) überschichtet um einen cytopathischen Effekt von annähernd 90 % innerhalb 36 h zu erreichen und erneut inkubiert. Nach 24 h und mikroskopischer Kontrolle wurden die Zellen mit Methylviolett angefärbt.

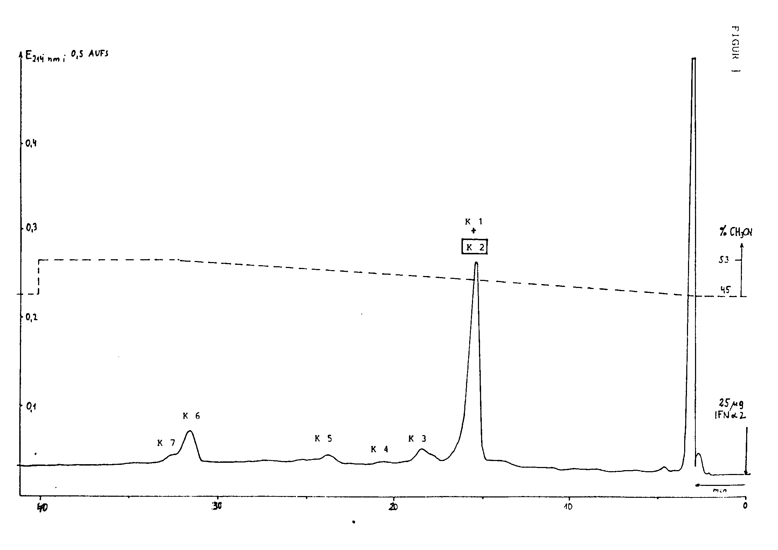

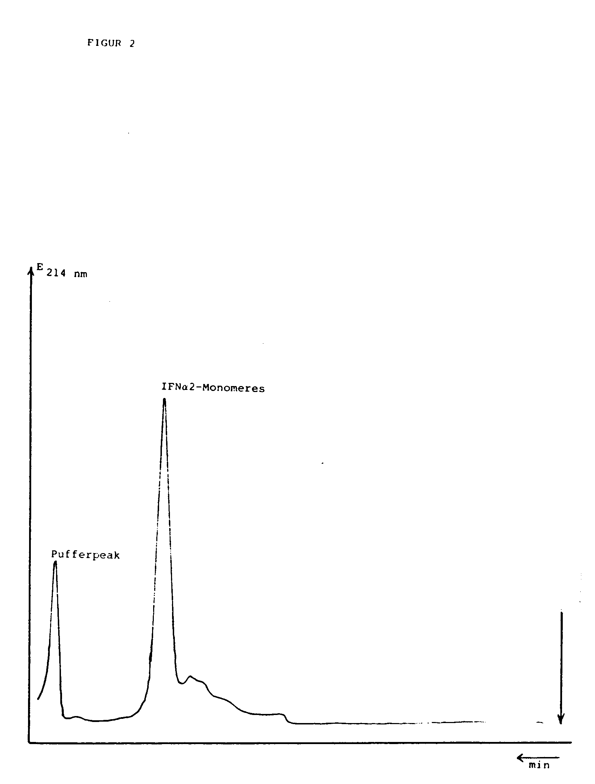

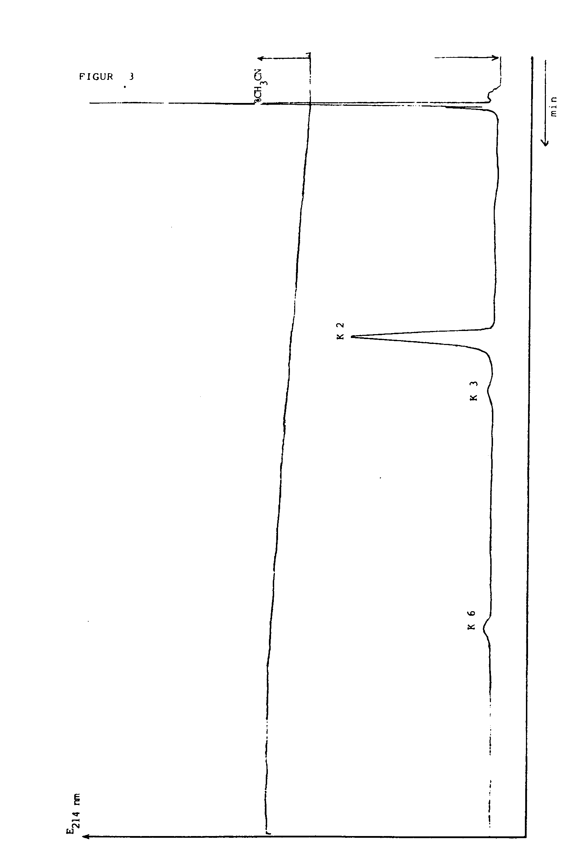

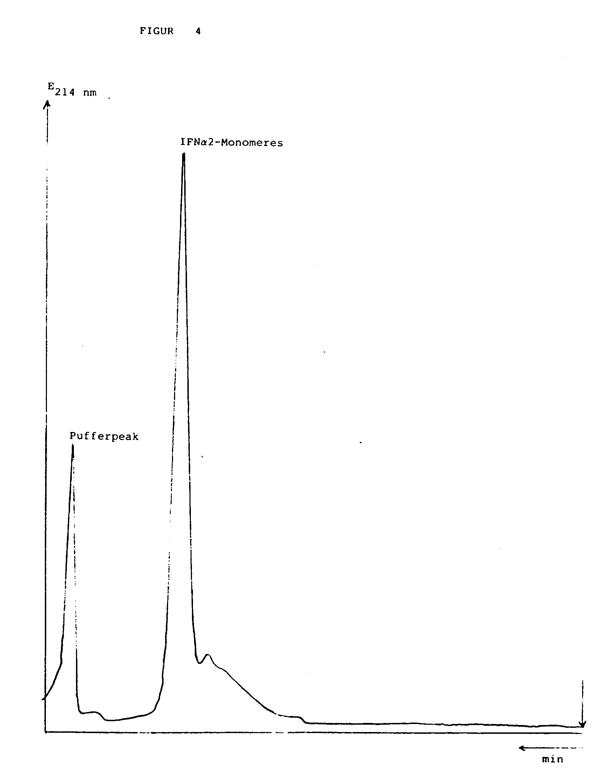

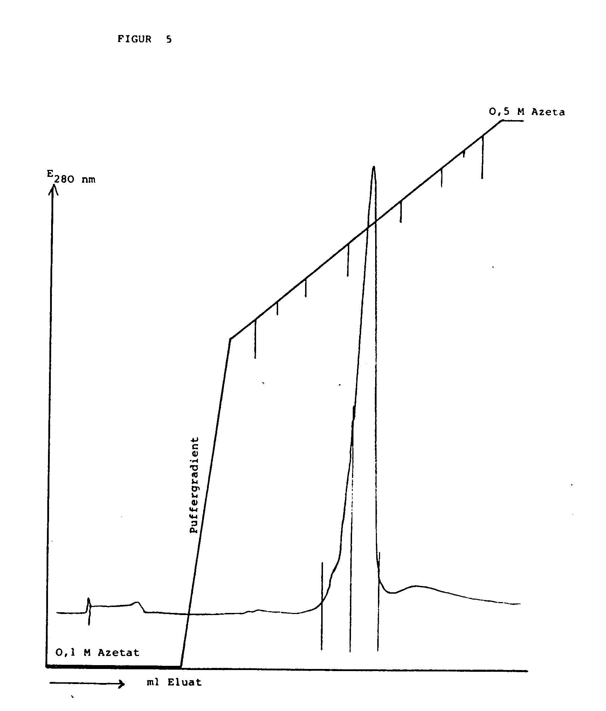

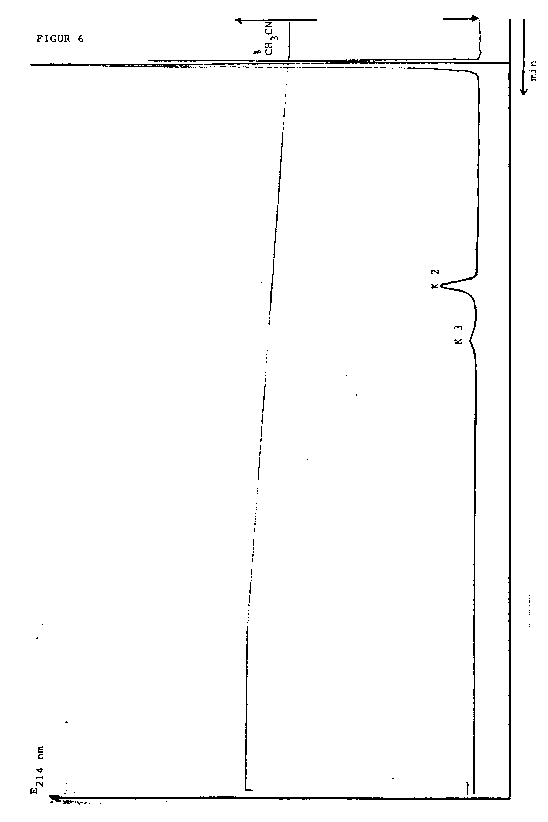

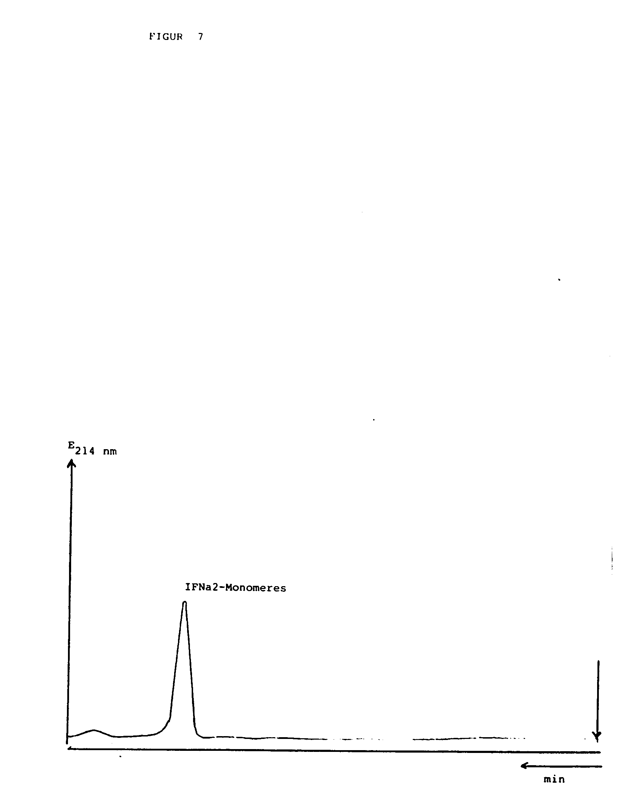

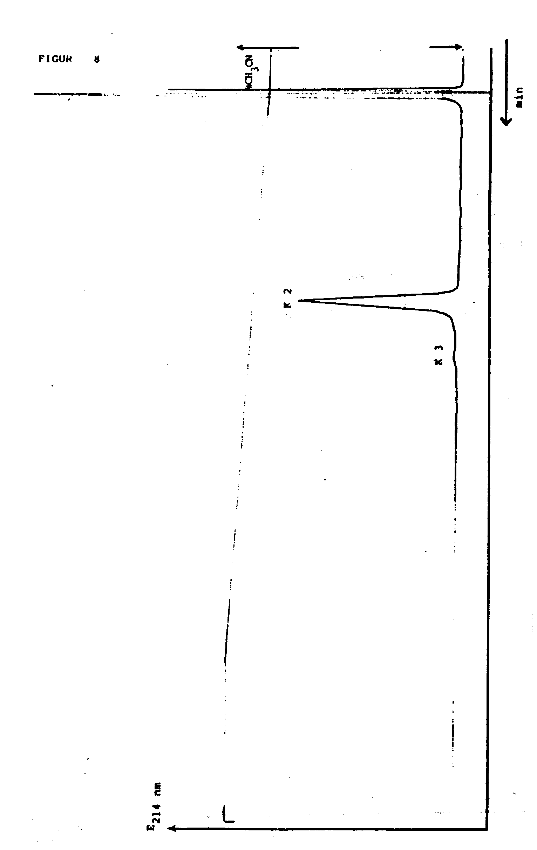

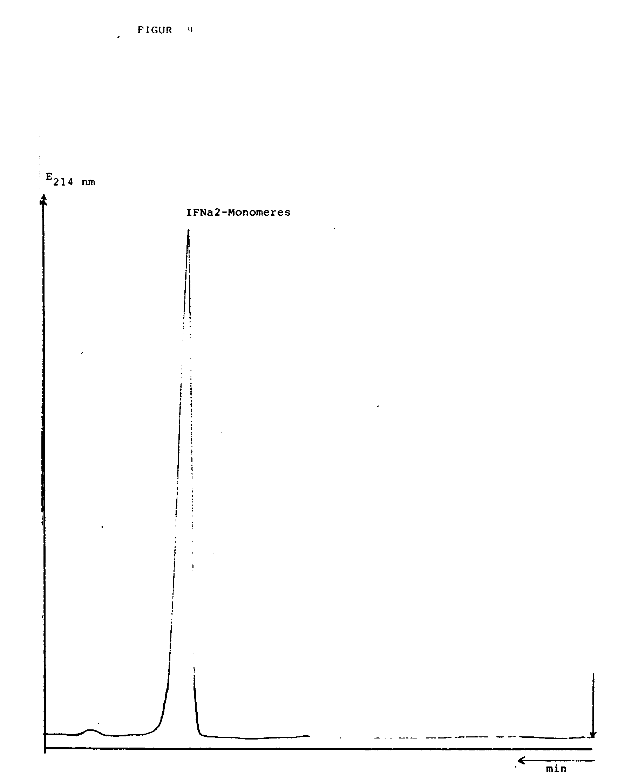

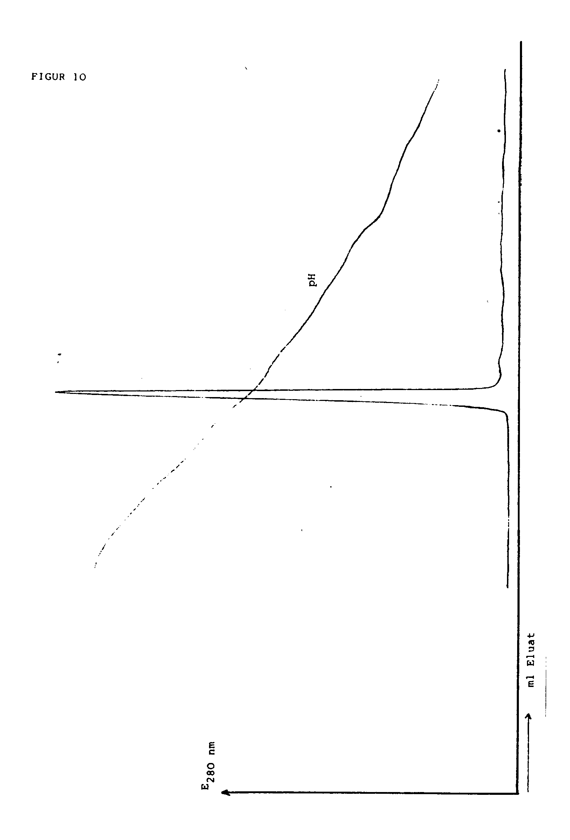

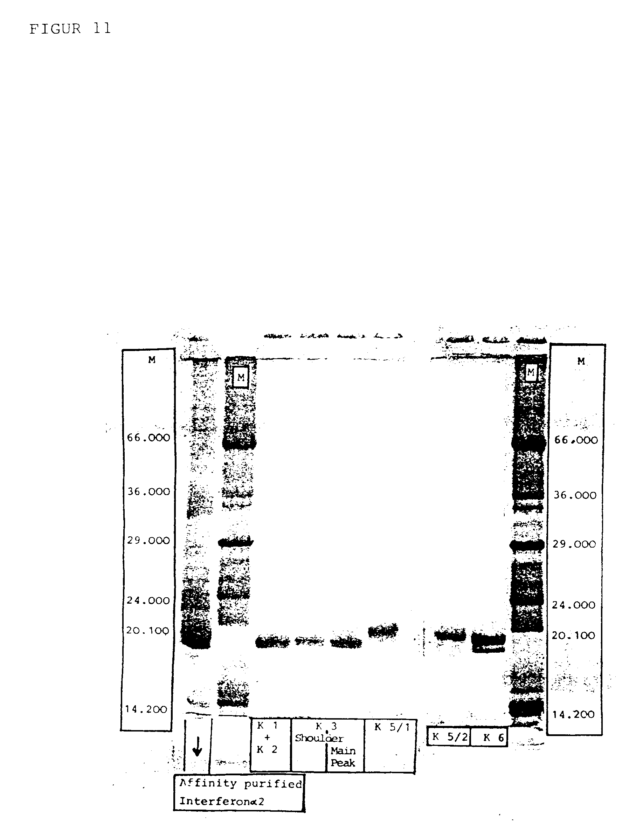

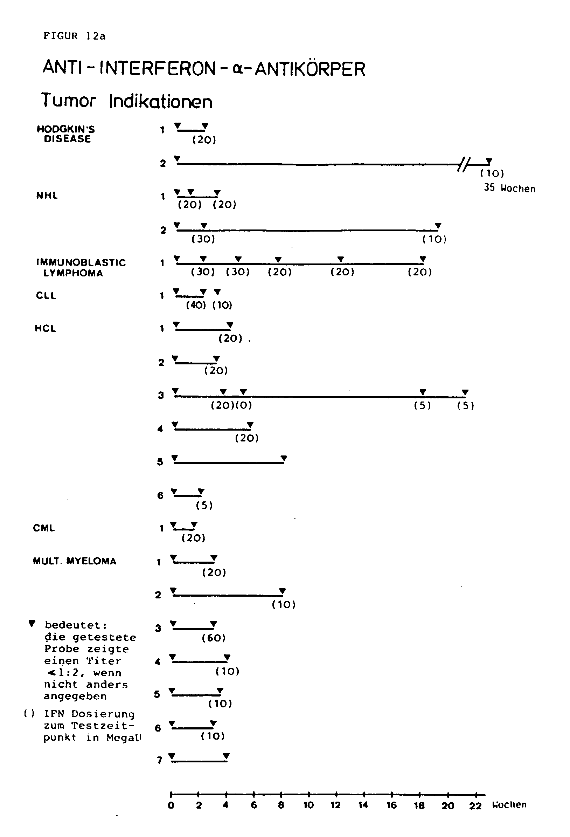

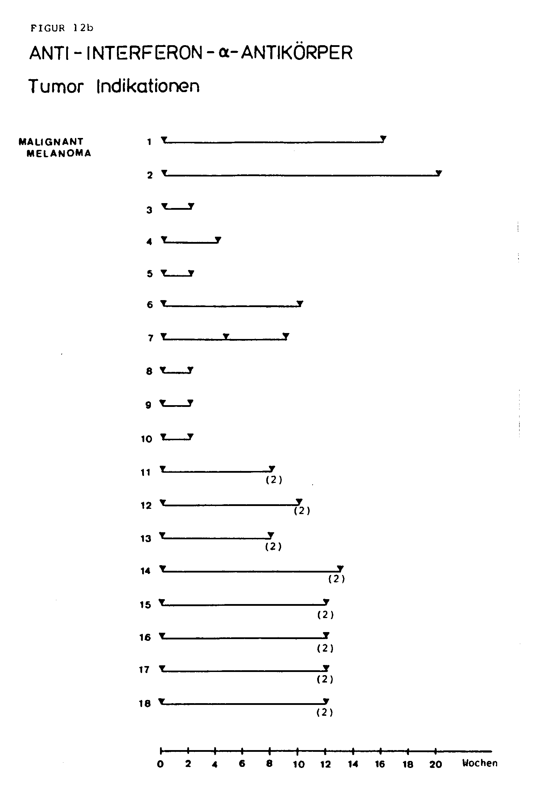

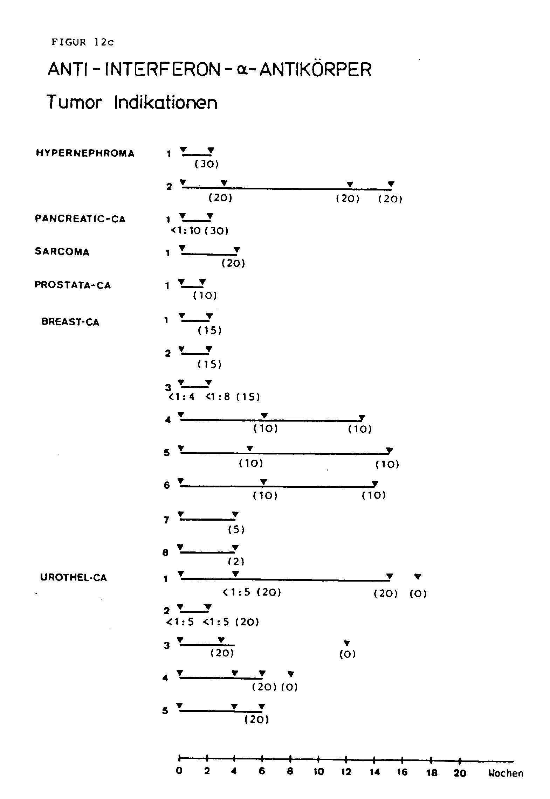

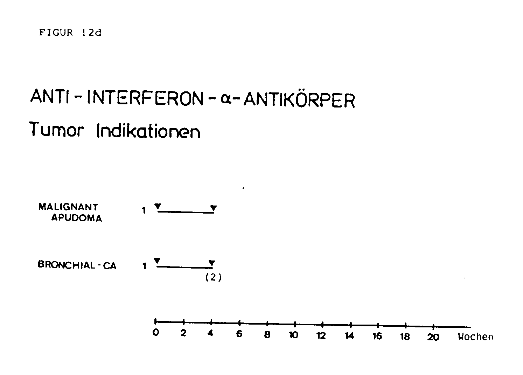

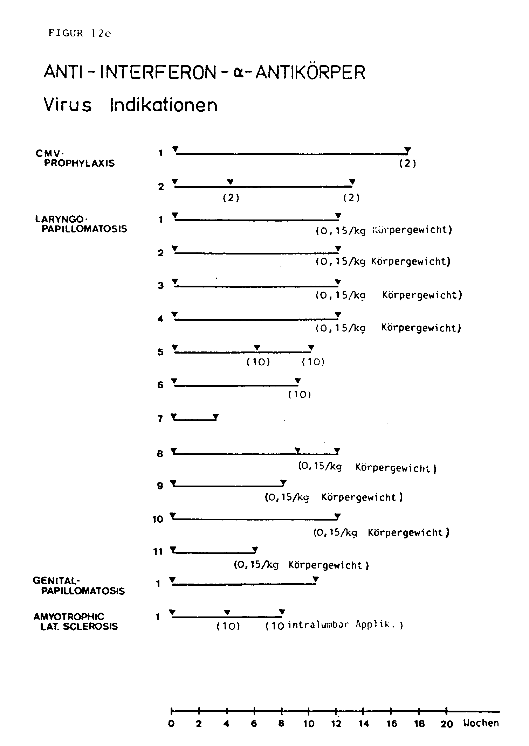

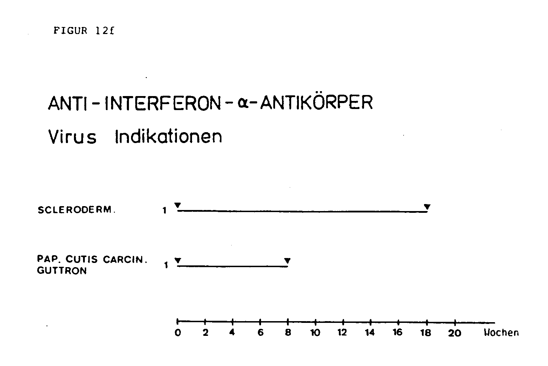

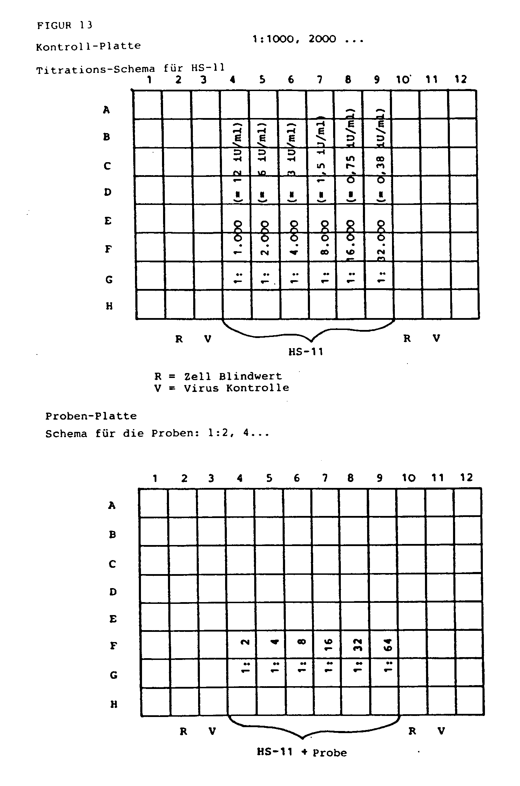

Die Ergebnisse sind in den Figuren 12a - 12f gezeigt; das Schema findet sich im Anhang.

METHODIK

Zur Analytik wurden folgende Verfahren verwendet:

Proteinbestimmung

BIORAD PROTEIN ASSAY: Dieser Assay verwendet den Farbstoff Coomassie Brilliant Blue und mißt den Protein-Farbstoffkomplex bei 595 nm . Der verwendete Standard ist Rinderserumalbumin.

Planimetrische Bestimmung der bei 214 nm gemessenen Peakflächen, die bei der Gelpermeations-HPLC aufgezeichnet wurden. Die Umrechnung erfolgte mit Hilfe eines Faktors aus den Eichsubstanzen Rinderserumalbumin, Ovalbumin, Trypsinogen und Lysozym. Diese Bestimmung wurde vor allem bei den Präparaten nach der Reinigungsstufe Tandem-Chromatographie, pH 4,5 Fällung und FPLC un MONO-S zusätzlich oder ausschließlich vorgenommen.

Interferonbestimmung

Der von der Fa. CELLTECH (U.K.) käufliche "NK₂-IRMA" für humanes Interferon alpha wurde eingesetzt. Als Standard diente ein Laborstandard "HS 11", der mit Hilfe eines biologischen Assays (Plaquereduktionstest WISH-Zellen und Vesicular Stomatitis Virus) auf den Internationalen Standard B 69/19 eingestellt wurde.

SDS-Gelelektrophorese

Eingesetzt wurde die Methode von LAEMMLI (Nature. 227, 680,(1980)).Der zum Anfärben der Proteine verwendete Farbstoff war Coomassie Brilliant Blue. Bei den Reinheitskontrollen der Interferon-Präparate wurden jeweils 20 Mikrogramm eingesetzt.

Chromatofokussierung

Es wurde die Methode von Bodo und Adolf ("Separation and Characterization of Human IFN-alpha Subtypes", in "The Biology of the Interferon System, Seiten 113 - 118, Elsevier 1983, Edts.E.DeMaeyer and H.Schellekens) verwendet, bei der eine MONO-P Chromatofokussiersäule HR 5/20 (Pharmacia) in einem pH-Bereich von 4 - 7 eingesetzt wurde. Die Puffer enthielten anstelle des angegebenen Zusatzes von 25 % 1,2-Propandiol 25 % Acetonitril um die Flußgeschwindigkeit erhöhen zu können. Die Registrierung der Proteinkonzentration erfolgte bei 280 nm, die pH-Registrierung erfolgte automatisch. Die zu analysierenden Proben wurden lyophilisiert, in Wasser zu 1 mg/ml gelöst und sodann mit 5 Volumen Puffer A (pH 7,1) verdünnt. 0,2 - 1,0 mg Interferon je Analyse wurden eingesetzt.

EP 0 203 382 B1

Gelpermeations - HPLC (Hochdruckflüssigkeitschromatographie)

Stationäre Phase:

WATERS I-125; 2 x (300 mm x 7,8 mm);
10 $\mu$m Teilchendurchmesser

Mobile Phase:

0,5 M $Na_2SO_4$
0,02 M $NaH_2PO_4$, auf pH 7,0 eingestellt mit NaOH
0,04 % Tween 20
25 % Propylenglykol

Flußgeschwindigkeit:

0,5 ml/min

Detektion:

UV-Absorption bei 214 nm

## Molekulargewichtseichung:

| | | |
|---|---|---|
| Bovine Serum Albumine | M | 66.000 |
| Ovalbumin | M | 45.000 |
| Trypsinogen | M | 24.000 |
| Lysozym | M | 14.300 |

Reverse Phase HPLC (Hochdruckflüssigkeitschromatographie)

Stationäre Phase:

Bakerbond WP C 18; 250 mm x 4,6 mm;
5 $\mu$m Teilchendurchmesser;
30 nm Porendurchmesser

Mobile Phase:

A: 0,1 % Trifluoressigsäure in Wasser, pH 2,2
B: 0,1 % Trifluoressigsäure in Acetonitril

Gradientenprogramm:

0 - 2 min: 45 % B
2 - 32 min: 45 - 53 % B
32 - 40 min: 53 % B
40 - 50 min: 45 % B

Flußgeschwindigkeit:

1 ml/min

18

Detektion:

UV-Absorption bei 214 nm

Legende zu den Abbildungen

| | |
|---|---|
| Figur 1: | Chromatogramm der Reverse-Phase HPLC des sauren Eluates nach der Tandem-Chromatographie: Darstellung der Komponenten K 1 - K 7. |
| Figur 2: | Chromatogramm der Gelpermeations-HPLC des sauren Eluates nach der Tandem-Chromatographie. |
| Figur 3: | Chromatogramm der Reverse-Phase HPLC nach der Fällung bei pH 4,5: Darstellung der Komponenten K 1, K 2, K 3 und K 6. |
| Figur 4: | Chromatogramm der Gelpermeations-HPLC nach der Fällung bei pH 4,5. |
| Figur 5: | Chromatogramm der FPLC an MONO-S bei pH 4,5 mit Hilfe eines Ammonazetat-Gradienten von 0,1 - 0,5 M |
| Figur 6: | Chromatogramm der Reverse-Phase HPLC der "Schulter-Fraktion" des MONO-S - Peaks |
| Figur 7: | Chromatogramm der Gelpermeations-HPLC der "Schulter-Fraktion" des MONO-S - Peaks |
| Figur 8: | Chromatogramm der Reverse-Phase HPLC der "Haupt-Fraktion" des MONO-S - Peaks |
| Figur 9: | Chromatogramm der Gelpermeations-HPLC der "Haupt-Fraktion" des MONO-S - Peaks |
| Figur 10: | Chromatogramm der Chromatofokussierung der "Haupt-Fraktion" des MONO-S - Peaks |
| Figur 11: | Photo der Gelelektrophorese des sauren Eluates nach der Tandem-Chromatographie sowie der durch Reverse-Phase HPLC getrennten Komponenten K 1 - K 7. |
| Figur 12a - 12f: | Ergebnisse der Anti-IFN-$\alpha$-Antikörper; Untersuchungen bei verschiedenen Indikationen |

## Test-Typ: Neutralisations Assay

### (10 iU /ml IFN-$\alpha$/A-599/EMC)

| | |
|---|---|
| **Anzahl Patienten insgesamt:** | **75** |
| **Anzahl Patienten mit Tumor Indikationen:** | **58** |
| **Anzahl Patienten ohne stimulierte Antikörper** | **58** |
| **Anzahl Patienten mit Virus Indikationen:** | **17** |
| **Anzahl Patienten ohne stimulierte Antikörper** | **17** |

| | |
|---|---|
| Figur 13: | Schema für den Anti-Interferon-$\alpha$-Antikörper Assay |
| Tabelle 1: | Übersicht über die Reinigung |
| Tabelle 2: | Beurteilung der Reinheit durch HPLC |

Tabelle    1

| Reinigungsstufe | Vol (ml) | mg Protein | IFN (I.E.)* | I.E.IFN/mg Protein | % Ausbeute |
|---|---|---|---|---|---|
| Rohextrakt | 2540 | 5330 | $17,1 \times 10^9$ | $3,21 \times 10^6$ | 100 |
| Ammonsulfat Überstand | 3120 | n.b.* | $0,12 \times 10^9$ | | 0,7 |
| Pellet nach Lösen + Dialyse | 282 | n.b.* | $13,3 \times 10^9$ | | 77,6 |
| Eluat der Tandem-Chromatographie | 16,8 | 54,4 | $12,3 \times 10^9$ | $226 \times 10^6$ | 71,9 |
| Überstand der pH 4,5-Fällung | 18,3 | 46,3 | $11,8 \times 10^9$ | $255 \times 10^6$ | 69,0 |
| FPLC an Mono-S | | | | | |
| a) "Schulter"-Fraktion | 9,1 | 4,1 | $1,33 \times 10^9$ | $324 \times 10^6$ | 7,7 |
| b) Haupt-Fraktion | 9,8 | 16,1 | $5,18 \times 10^9$ | $322 \times 10^6$ | 30,3 |

*
n.b. = nicht bestimmt
I.E. = Internationale Einheiten

EP 0 203 382 B1

Tabelle 2

|  | IFN (mg/ml) | Gelpermeations-HPLC | | Reverse-Phase-HPLC ** | | | | | |
|  |  | Reinheit des Monomeren (MG 19.000) | MG >70.000 | K 2 + 1 | K3 | K4 | K5 | K6 | K7 |
|---|---|---|---|---|---|---|---|---|---|
| Saures Eluat nach Tandem-Chromatographie | 2,14 | 74 % |  | 100 | 7,0 | n.n.* | 1,6 | 22,8 | ~1 |
| Überstand nach Fällung bei pH 4,5 | 2,0 | 79 % |  | 100 | 4,0 | n.n. | <1 | 5,4 | n.n. |
| nach FPLC an MONO-S a) Schulter-Frakt. | 0,45 | >99 % | <0,2 % | 100 | 16,0 | n.n. | n.n. | n.n. | n.n. |
| b) Haupt-Frakt. | 1,64 | >99 % | <0,2 % | 100 | 1,2 | n.n. | n.n. | n.n. | n.n. |

* = nicht nachweisbar

** = Relative Peak-Höhen (214 nm), K 2 + 1 = 100%

## Patentansprüche

1. Verfahren zur Herstellung von rekombinantem α-Interferon, dadurch gekennzeichnet, daß der das Interferongen enthaltende Wirtsorganismus unter üblichen Bedingungen kultiviert wird,

21

die Zellen nach einer Wachstumszeit, in der nicht mehr als 20% Methionin-Interferon gebildet wird, abgetötet und die Zellen geerntet werden,

das exprimierte Interferon in üblicher Weise abgetrennt wird,

Zelltrümmer im schwach alkalischen abgetrennt werden,

das Interferon konzentriert und durch eine Tandem-Chromatographie vorgereinigt wird,

das Eluat zur Abtrennung von Verunreinigungen auf pH 4,0 - 4,8 eingestellt wird,

das Interferon durch Chromatographie an einer Kationenaustauschersäule mit einem flüchtigen Puffer als Elutionsmittel bei pH-Werten von 4,0 - 5,0 endgereinigt und anschließend lyophilisiert wird.

2. Verfahren nach Anspruch 1, dadurch gekennzeichnet, daß die Tandem-Chromatographie aus einer Zellulose-Vorsäule und einer Affinitätschromatographie besteht, und daß die zu reinigende Substanz über beide Säulen mit einer geeigneten Waschlösung gewaschen und das α-Interferon anschließend mit einem geeigneten Elutionsmittel von der Affinitätssäule eluiert wird.

3. Verfahren nach einem der vorhergehenden Ansprüche, dadurch gekennzeichnet, daß das Eluat der Tandem-Chromatographie zur Abtrennung von Verunreinigungen auf pH 4,5 eingestellt wird.

4. Verfahren nach einem der vorhergehenden Ansprüche zur Herstellung eines rekombinanten, homogenen, reinen α-Interferons, das 0 - 5 % Methionin-Interferon aufweist, das frei von reduzierten Formen und Bruchstücken des Interferons ist, das 0 - 0,2 % Oligomere und 0 - 2 % Dimere/Trimere/Tetramere enthält und bei dem 90 - 100 % des Monomerengehaltes aus dem α-Interferon mit je einer Disulfidbrücke zwischen den Cysteinen an Position 1 und 98 und 29 und 138 besteht.

5. Verfahren nach einem der vorhergehenden Ansprüche zur Herstellung eines rekombinanten, homogenen, reinen Human-α-Interferons der Aminosäuresequenz

```
Cys Asp Leu Pro Gln Thr His Ser Leu Gly Ser Arg Arg
Thr Leu Met Leu Leu Ala Gln Met Arg Arg Ile Ser Leu
Phe Ser Cys Leu Lys Asp Arg Arg Asp Phe Gly Phe Pro
Gln Glu Glu Phe Gly Asn Gln Phe Gln Lys Ala Glu Thr
Ile Pro Val Leu His Glu Met Ile Gln Gln Ile Phe Asn
Leu Phe Ser Thr Lys Asp Ser Ser Ala Ala Trp Asp Glu
Thr Leu Leu Asp Lys Phe Tyr Thr Glu Leu Tyr Gln Gln
Leu Asn Asp Leu Glu Ala Cys Val Ile Gln Gly Val Gly
Val Thr Glu Thr Pro Leu Met Lys Glu Asp Ser Ile Leu
Ala Val Arg Lys Tyr Phe Gln Arg Ile Thr Leu Tyr Leu
Lys Glu Lys Lys Tyr Ser Pro Cys Ala Trp Glu Val Val
Arg Ala Glu Ile Met Arg Ser Phe Ser Leu Ser Thr Asn
Leu Gln Glu Ser Leu Arg Ser Lys Glu
```

das 0 - 5 % Methionin-Interferon aufweist,

das frei von reduzierten Formen und Bruchstücken des α-Interferons ist, das 0 - 0,2 % Oligomere und 0 - 2 % Dimere/Trimere/Tetramere und bei dem das α-Interferon je eine Disulfidbrücke zwischen den Cysteinen an Position 1 und 98 und 29 und 138 aufweist.

6. Verfahren nach einem der vorhergehenden Ansprüche zur Herstellung eines festen α-Interferons.

7. Verfahren nach einem der vorhergehenden Ansprüche zur Herstellung eines nichtimmunogenen α-Interferons.

**8.** Rekombinantes α-Interferon, dadurch gekennzeichnet, daß es der Aminosäuresequenz

```
Cys Asp Leu Pro Gln Thr His Ser Leu Gly Ser Arg Arg
Thr Leu Met Leu Leu Ala Gln Met Arg Arg Ile Ser Leu
Phe Ser Cys Leu Lys Asp Arg Arg Asp Phe Gly Phe Pro
Gln Glu Glu Phe Gly Asn Gln Phe Gln Lys Ala Glu Thr
Ile Pro Val Leu His Glu Met Ile Gln Gln Ile Phe Asn
Leu Phe Ser Thr Lys Asp Ser Ser Ala Ala Trp Asp Glu
Thr Leu Leu Asp Lys Phe Tyr Thr Glu Leu Tyr Gln Gln
Leu Asn Asp Leu Glu Ala Cys Val Ile Gln Gly Val Gly
Val Thr Glu Thr Pro Leu Met Lys Glu Asp Ser Ile Leu
Ala Val Arg Lys Tyr Phe Gln Arg Ile Thr Leu Tyr Leu
Lys Glu Lys Lys Tyr Ser Pro Cys Ala Trp Glu Val Val
Arg Ala Glu Ile Met Arg Ser Phe Ser Leu Ser Thr Asn
Leu Gln Glu Ser Leu Arg Ser Lys Glu
```

entspricht.

**9.** Rekombinantes α-Interferon, das der Aminosäuresequenz

```
Cys Asp Leu Pro Gln Thr His Ser Leu Gly Ser Arg Arg
Thr Leu Met Leu Leu Ala Gln Met Arg Arg Ile Ser Leu
Phe Ser Cys Leu Lys Asp Arg Arg Asp Phe Gly Phe Pro
Gln Glu Glu Phe Gly Asn Gln Phe Gln Lys Ala Glu Thr
Ile Pro Val Leu His Glu Met Ile Gln Gln Ile Phe Asn
Leu Phe Ser Thr Lys Asp Ser Ser Ala Ala Trp Asp Glu
Thr Leu Leu Asp Lys Phe Tyr Thr Glu Leu Tyr Gln Gln
Leu Asn Asp Leu Glu Ala Cys Val Ile Gln Gly Val Gly
Val Thr Glu Thr Pro Leu Met Lys Glu Asp Ser Ile Leu
Ala Val Arg Lys Tyr Phe Gln Arg Ile Thr Leu Tyr Leu
Lys Glu Lys Lys Tyr Ser Pro Cys Ala Trp Glu Val Val
Arg Ala Glu Ile Met Arg Ser Phe Ser Leu Ser Thr Asn
Leu Gln Glu Ser Leu Arg Ser Lys Glu
```

entspricht, in homogener, reiner Form vorliegt, mit 0 - 5 % Methionin-Interferon, 0 - 0,2 % Oligomeren und 0 - 2 % Dimeren/Trimeren/Tetrameren, das frei von reduzierten Formen und Bruchstücken des α-Interferons ist und bei dem 90 - 100 % des Monomerengehaltes aus de α-Interferon mit je einer Disulfidbrücke zwischen den Cysteinen an Position 1 und 98 und 29 und 138 besteht.

**10.** Humanes α-Interferon nach einem der Ansprüche 8 und 9.

**11.** α-Interferon nach einem der Ansprüche 8 bis 10 zur Verwendung bei der therapeutischen Behandlung viraler und tumoraler Erkrankungen.

12. Pharmazeutisches Mittel zur therapeutischen Behandlung, dadurch gekennzeichnet, daß es ein α-Interferon nach einem der Ansprüche 8 bis 10 und eine oder mehrere pharmazeutische, inerte Hilfs- und/oder Trägerstoffe enthält.

## Claims

1. Process for the preparation of recombinant α-interferon, characterised in that
the host organism containing the interferon gene is cultivated under conventional conditions,
after a growth period in which not more than 20% methionine interferon is formed the cells are killed off and harvested,
the expressed interferon is removed in conventional manner,
the cell debris is removed in a slightly alkaline medium,
the interferon is concentrated and subjected to preliminary purification by tandem chromatography,
the eluate is adjusted to pH 4.0-4.8 to remove any impurities,
the interferon is finally purified by chromatography on a cation exchanger column with a volatile buffer as eluant at pH levels from 4.0 to 5.0 and is then lyophilised.

2. Process according to claim 1, characterised in that the tandem chromatography consists of a preliminary cellulose column and affinity chromatography and in that the substance to be purified is washed through both columns with a suitable washing solution and the α-interferon is subsequently eluted from the affinity column with a suitable eluant.

3. Process according to one of the preceding claims, characterised in that the eluate from the tandem chromatography is adjusted to pH 4.5 to remove any impurities.

4. Process according to one of the preceding claims for the preparation of a recombinant, homogeneous, pure α-interferon which contains 0 to 5% methionine interferon, which is free from reduced forms and fragments of interferon, which contains 0 to 0.2% oligomer and 0 to 2% dimer/trimer/tetramer and in which 90 to 100% of the monomer content consists of the α-interferon with disulphide bridges between the cysteines at positions 1 and 98 and 29 and 138.

5. Process according to one of the preceding claims for the preparation of a recombinant, homogeneous, pure, human α-interferon with the amino acid sequence

```
Cys Asp Leu Pro Gln Thr His Ser Leu Gly Ser Arg Arg
Thr Leu Met Leu Leu Ala Gln Met Arg Arg Ile Ser Leu
Phe Ser Cys Leu Lys Asp Arg Arg Asp Phe Gly Phe Pro
Gln Glu Glu Phe Gly Asn Gln Phe Gln Lys Ala Glu Thr
Ile Pro Val Leu His Glu Met Ile Gln Gln Ile Phe Asn
Leu Phe Ser Thr Lys Asp Ser Ser Ala Ala Trp Asp Glu
Thr Leu Leu Asp Lys Phe Tyr Thr Glu Leu Tyr Gln Gln
Leu Asn Asp Leu Glu Ala Cys Val Ile Gln Gly Val Gly
Val Thr Glu Thr Pro Leu Met Lys Glu Asp Ser Ile Leu
Ala Val Arg Lys Tyr Phe Gln Arg Ile Thr Leu Tyr Leu
Lys Glu Lys Lys Tyr Ser Pro Cys Ala Trp Glu Val Val
Arg Ala Glu Ile Met Arg Ser Phe Ser Leu Ser Thr Asn
Leu Gln Glu Ser Leu Arg Ser Lys Glu
```

which contains 0 to 5% methionine-interferon, which is free from reduced forms and fragments of α-interferon, which contains 0 - 0.2% oligomer and 0 to 2% dimer/trimer/tetramer and wherein the α-interferon contains disulphide bridges between the cysteines at positions 1 and 98 and 29 and 138.

6. Process according to one of the preceding claims for preparing a solid α-interferon.

7. Process according to one of the preceding claims for preparing a non-immunogenic α-interferon.

8. Recombinant α-interferon, characterised in that it corresponds to the amino acid sequence

```
Cys Asp Leu Pro Gln Thr His Ser Leu Gly Ser Arg Arg
Thr Leu Met Leu Leu Ala Gln Met Arg Arg Ile Ser Leu
Phe Ser Cys Leu Lys Asp Arg Arg Asp Phe Gly Phe Pro
Gln Glu Glu Phe Gly Asn Gln Phe Gln Lys Ala Glu Thr
Ile Pro Val Leu His Glu Met Ile Gln Gln Ile Phe Asn
Leu Phe Ser Thr Lys Asp Ser Ser Ala Ala Trp Asp Glu
Thr Leu Leu Asp Lys Phe Tyr Thr Glu Leu Tyr Gln Gln
Leu Asn Asp Leu Glu Ala Cys Val Ile Gln Gly Val Gly
Val Thr Glu Thr Pro Leu Met Lys Glu Asp Ser Ile Leu
Ala Val Arg Lys Tyr Phe Gln Arg Ile Thr Leu Tyr Leu
Lys Glu Lys Lys Tyr Ser Pro Cys Ala Trp Glu Val Val
Arg Ala Glu Ile Met Arg Ser Phe Ser Leu Ser Thr Asn
Leu Gln Glu Ser Leu Arg Ser Lys Glu.
```

9. Recombinant α-interferon, which corresponds to the amino acid sequence

```
Cys Asp Leu Pro Gln Thr His Ser Leu Gly Ser Arg Arg
Thr Leu Met Leu Leu Ala Gln Met Arg Arg Ile Ser Leu
Phe Ser Cys Leu Lys Asp Arg Arg Asp Phe Gly Phe Pro
Gln Glu Glu Phe Gly Asn Gln Phe Gln Lys Ala Glu Thr
Ile Pro Val Leu His Glu Met Ile Gln Gln Ile Phe Asn
Leu Phe Ser Thr Lys Asp Ser Ser Ala Ala Trp Asp Glu
Thr Leu Leu Asp Lys Phe Tyr Thr Glu Leu Tyr Gln Gln
Leu Asn Asp Leu Glu Ala Cys Val Ile Gln Gly Val Gly
Val Thr Glu Thr Pro Leu Met Lys Glu Asp Ser Ile Leu
Ala Val Arg Lys Tyr Phe Gln Arg Ile Thr Leu Tyr Leu
Lys Glu Lys Lys Tyr Ser Pro Cys Ala Trp Glu Val Val
Arg Ala Glu Ile Met Arg Ser Phe Ser Leu Ser Thr Asn
Leu Gln Glu Ser Leu Arg Ser Lys Glu
```

is present in a homogeneous, pure form, with 0 - 5% methionine interferon, 0 - 0.2% oligomers and 0 - 2% dimers/trimers/tetramers, is free from reduced forms and fragments of the α-interferon and wherein 90 - 100% of the monomer content consists of the α-interferon with disulphide bridges between the cysteines at positions 1 and 98 and 29 and 138.

**10.** Human α-interferon according to one of claims 8 and 9.

**11.** α-Interferon according to one of claims 8 to 10 for use in the therapeutic treatment of viral and tumoral diseases.

**12.** Pharmaceutical composition for therapeutic treatment, characterised in that it contains an α-interferon according to one of claims 8 to 10 and one or more pharmaceutical, inert excipients and/or carriers.

**Revendications**

**1.** Procédé de préparation d'interféron α recombinant, caractérisé en ce que
l'organisme hôte qui contient le gène de l'interféron est cultivé dans des conditions habituelles,
les cellules sont tuées au bout d'une durée de croissance pendant laquelle il ne se forme pas plus de 20% d'interféron-méthionine et les cellules sont récoltées,
l'interféron exprimé est séparé de manière habituelle,
les débris cellulaires sont séparés en milieu faiblement alcalin,
l'interféron est concentré et prépurifié par chromatographie en tandem,
l'éluat est amené à pH 4,0-4,8 pour la séparation des impuretés,
l'interféron est soumis à une purification finale par chromatographie sur une colonne d'échangeur de cations avec comme éluant un tampon volatil à des pH de 4,0 - 5,0 puis il est lyophilisé.

**2.** Procédé selon la revendication 1, caractérisé en ce que la chromatographie en tandem consiste en une précolonne de cellulose et en une chromatographie d'affinité, et en ce que la substance à purifier est lavée sur les deux colonnes avec une solution de lavage appropriée et l'interféron α est ensuite élué de la colonne d'affinité avec un éluant approprié.

**3.** Procédé selon l'une des revendications précédentes, caractérisé en ce que l'éluat de la chromatographie en tandem est amené à pH 4,5 pour la séparation des impuretés.

**4.** Procédé selon l'une des revendications précédentes pour la préparation d'un interféron α recombinant, homogène, pur, qui présente 0 à 5% d'interféron-méthionine, qui est exempt de formes réduites et de fragments de l'interféron, qui contient 0 à 0,2% d'oligomères et 0 à 2% de dimères/trimères/tétramères et dans lequel 90 à 100% de la teneur en monomères consiste en l'interféron α avec un pont disulfure entre les cystéines aux positions 1 et 98 et entre les cystéines aux positions 29 et 138.

**5.** Procédé selon l'une des revendications précédentes pour la préparation d'un interféron α humain recombinant, homogène, pur de séquence d'acides aminés

```
Cys Asp Leu Pro Gln Thr His Ser Leu Gly Ser Arg Arg
Thr Leu Met Leu Leu Ala Gln Met Arg Arg Ile Ser Leu
Phe Ser Cys Leu Lys Asp Arg Arg Asp Phe Gly Phe Pro
Gln Glu Glu Phe Gly Asn Gln Phe Gln Lys Ala Glu Thr
Ile Pro Val Leu His Glu Met Ile Gln Gln Ile Phe Asn
Leu Phe Ser Thr Lys Asp Ser Ser Ala Ala Trp Asp Glu
Thr Leu Leu Asp Lys Phe Tyr Thr Glu Leu Tyr Gln Gln
Leu Asn Asp Leu Glu Ala Cys Val Ile Gln Gly Val Gly
Val Thr Glu Thr Pro Leu Met Lys Glu Asp Ser Ile Leu
Ala Val Arg Lys Tyr Phe Gln Arg Ile Thr Leu Tyr Leu
Lys Glu Lys Lys Tyr Ser Pro Cys Ala Trp Glu Val Val
Arg Ala Glu Ile Met Arg Ser Phe Ser Leu Ser Thr Asn
Leu Gln Glu Ser Leu Arg Ser Lys Glu
```

qui présente 0 à 5% d'interféron-méthionine, qui est exempt de formes réduites et de fragments de l'interféron $\alpha$, qui contient 0 à 0,2% d'oligomères et 0 à 2% de dimères/trimères/tétramères et dans lequel l'interféron $\alpha$ présente un pont disulfure entre les cystéines aux positions 1 et 98 et entre les cystéines aux positions 29 et 138.

6. Procédé selon l'une des revendications précédentes pour la préparation d'un interféron $\alpha$ solide.

7. Procédé selon l'une des revendications précédentes pour la préparation d'un interféron $\alpha$ non immunogène.

8. Interféron $\alpha$ recombinant, caractérisé en ce qu'il correspond à la séquence d'acides aminés

```
Cys Asp Leu Pro Gln Thr His Ser Leu Gly Ser Arg Arg
Thr Leu Met Leu Leu Ala Gln Met Arg Arg Ile Ser Leu
Phe Ser Cys Leu Lys Asp Arg Arg Asp Phe Gly Phe Pro
Gln Glu Glu Phe Gly Asn Gln Phe Gln Lys Ala Glu Thr
Ile Pro Val Leu His Glu Met Ile Gln Gln Ile Phe Asn
Leu Phe Ser Thr Lys Asp Ser Ser Ala Ala Trp Asp Glu
Thr Leu Leu Asp Lys Phe Tyr Thr Glu Leu Tyr Gln Gln
Leu Asn Asp Leu Glu Ala Cys Val Ile Gln Gly Val Gly
Val Thr Glu Thr Pro Leu Met Lys Glu Asp Ser Ile Leu
Ala Val Arg Lys Tyr Phe Gln Arg Ile Thr Leu Tyr Leu
Lys Glu Lys Lys Tyr Ser Pro Cys Ala Trp Glu Val Val
Arg Ala Glu Ile Met Arg Ser Phe Ser Leu Ser Thr Asn
Leu Gln Glu Ser Leu Arg Ser Lys Glu.
```

9. Interféron α recombinant, qui correspond à la séquence d'acides aminés

```
Cys Asp Leu Pro Gln Thr His Ser Leu Gly Ser Arg Arg
Thr Leu Met Leu Leu Ala Gln Met Arg Arg Ile Ser Leu
Phe Ser Cys Leu Lys Asp Arg Arg Asp Phe Gly Phe Pro
Gln Glu Glu Phe Gly Asn Gln Phe Gln Lys Ala Glu Thr
Ile Pro Val Leu His Glu Met Ile Gln Gln Ile Phe Asn
Leu Phe Ser Thr Lys Asp Ser Ser Ala Ala Trp Asp Glu
Thr Leu Leu Asp Lys Phe Tyr Thr Glu Leu Tyr Gln Gln
Leu Asn Asp Leu Glu Ala Cys Val Ile Gln Gly Val Gly
Val Thr Glu Thr Pro Leu Met Lys Glu Asp Ser Ile Leu
Ala Val Arg Lys Tyr Phe Gln Arg Ile Thr Leu Tyr Leu
Lys Glu Lys Lys Tyr Ser Pro Cys Ala Trp Glu Val Val
Arg Ala Glu Ile Met Arg Ser Phe Ser Leu Ser Thr Asn
Leu Gln Glu Ser Leu Arg Ser Lys Glu
```

qui est sous forme homogène, pure, avec 0 à 5% d'interféronméthionine, 0 à 0,2% d'oligomères et 0 à 2% de dimères/trimères/tétramères, qui est exempt de formes réduites et de fragments de l'interféron α et dans lequel 90 à 100% de la teneur en monomères consiste en l'interféron α avec un pont disulfure entre les cystéines aux positions 1 et 98 et entre les cystéines aux positions 29 et 138.

10. Interféron α humain selon l'une des revendications 8 et 9.

11. Interféron α selon l'une des revendications 8 à 10 destiné à être utilisé dans le traitement thérapeutique des maladies virales et tumorales.

12. Agent pharmaceutique pour traitement thérapeutique, caractérisé en ce qu'il contient un interféron α selon l'une des revendications 8 à 10 et un ou plusieurs adjuvants et/ou véhicules pharmaceutiques inertes.

FIGUR I

EP 0 203 382 B1

FIGUR 2

FIGUR 3

FIGUR 4

FIGUR 5

FIGUR 6

FIGUR   7

E$_{214}$ nm

IFNa2-Monomeres

min

FIGUR 8

FIGUR 9

$E_{214\ nm}$

IFNa2-Monomeres

min

EP 0 203 382 B1

FIGUR 1O

pH

E$_{280}$ nm

ml Eluat

38

FIGUR 11

FIGUR 12a

# ANTI - INTERFERON - α- ANTIKÖRPER

## Tumor Indikationen

FIGUR 12b

# ANTI - INTERFERON - α - ANTIKÖRPER

## Tumor Indikationen

MALIGNANT
MELANOMA

0 2 4 6 8 10 12 14 16 18 20 Wochen

FIGUR 12c

# ANTI - INTERFERON - α- ANTIKÖRPER

## Tumor Indikationen

FIGUR 12d

# ANTI - INTERFERON - α- ANTIKÖRPER

## Tumor Indikationen

FIGUR 12e

# ANTI - INTERFERON - α- ANTIKÖRPER

## Virus  Indikationen

FIGUR 12f

# ANTI - INTERFERON - α - ANTIKÖRPER

## Virus Indikationen

SCLERODERM.

PAP. CUTIS CARCIN.
GUTTRON

```
0  2  4  6  8  10  12  14  16  18  20   Wochen
```

EP 0 203 382 B1

FIGUR 13

Kontroll-Platte

1:1000, 2000 ...

Titrations-Schema für HS-11

R = Zell Blindwert
V = Virus Kontrolle

Proben-Platte

Schema für die Proben: 1:2, 4...

46